(19) Europäisches Patentamt
European Patent Office
Office européen des brevets

(11) EP 4 282 488 A1

(12) EUROPEAN PATENT APPLICATION
published in accordance with Art. 153(4) EPC

(43) Date of publication:
29.11.2023 Bulletin 2023/48

(21) Application number: 22759477.7

(22) Date of filing: 17.02.2022

(51) International Patent Classification (IPC):
A61P 37/08 (2006.01) A61P 43/00 (2006.01)
A61P 17/00 (2006.01) A61Q 19/08 (2006.01)
A61K 8/49 (2006.01) C12N 15/55 (2006.01)
C12N 15/63 (2006.01) C12Q 1/6851 (2018.01)
C12Q 1/686 (2018.01) C12Q 1/6897 (2018.01)
G01N 33/15 (2006.01) G01N 33/50 (2006.01)
G01N 33/53 (2006.01) G01N 33/68 (2006.01)
A61K 31/365 (2006.01)

(52) Cooperative Patent Classification (CPC):
A61K 8/49; A61K 31/365; A61P 17/00;
A61P 37/08; A61P 43/00; A61Q 19/08; C12N 9/14;
C12N 15/63; C12Q 1/6851; C12Q 1/686;
C12Q 1/6897; G01N 33/15; G01N 33/50;
G01N 33/53; G01N 33/68

(86) International application number:
PCT/JP2022/006379

(87) International publication number:
WO 2022/181438 (01.09.2022 Gazette 2022/35)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 26.02.2021 JP 2021031165

(71) Applicant: National University Corporation Kobe University
Kobe-shi, Hyogo 657-8501 (JP)

(72) Inventors:
- TSUJINO Yoshio
Kobe-shi Hyogo 657-8501 (JP)
- SATOH Ayano
Okayama-shi Okayama 700-8530 (JP)

(74) Representative: Page White Farrer
Bedford House
21a John Street
London WC1N 2BF (GB)

Remarks:
The complete document including Reference Table(s) and the Sequence Listing(s) can be downloaded from the EPO website

# (54) METHOD FOR EVALUATING XENOBIOTIC RESPONSE REACTION INDUCIBILITY OF SAMPLE OF INTEREST IN SKIN, METHOD FOR SEARCHING FOR SUBSTANCE CAPABLE OF INHIBITING XENOBIOTIC RESPONSE REACTION IN SKIN, AND AGENT CAPABLE OF INHIBITING XENOBIOTIC RESPONSE REACTION IN SKIN

(57) An object of the present invention is to provide a method for easily and efficiently evaluating the ability of test samples to trigger a foreign body reaction in the skin without using animals, a substance having an ability to inhibit a foreign body reaction in the skin, and a screening method for obtaining the substance. The above objects are attained by providing a method for evaluating an ability of a test sample to trigger a foreign body reaction in the skin, comprising: (1) a step of making a solution containing said test sample in contact with a keratinocyte transformed by a reporter vector comprising an enhancer sequence comprising a xenobiotic responsive element (XRE) and a reporter sequence encoding a reporter protein at downstream of said enhancer sequence, (2) a step of measuring an expression level of said reporter protein in said keratinocyte, and (3) a step of evaluating an ability of said test sample to trigger a foreign body reaction in the skin based on the measured expression level of said reporter protein; a method for screening a substance having an ability to inhibit a foreign body reaction in the skin



Processed by Luminess, 75001 PARIS (FR)

(Cont. next page)

using the evaluation method; and an agent for inhibiting a foreign body reaction in the skin comprising Fraglide-1, which was found using the screening method.

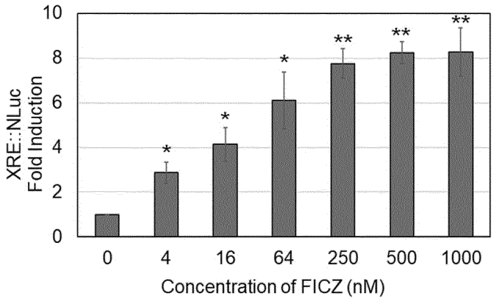


FIG. 3

## Description

### Technical Field

The present invention relates to a foreign body reaction in the skin. More particularly, the present invention relates to a method for evaluating an ability of a test sample to trigger a foreign body reaction in the skin, a method for screening a substance having an ability to inhibit a foreign body reaction in the skin, and an agent for inhibiting a foreign body reaction in the skin, which was found using the screening method.

### Background Art

The skin is an organ covering the whole body and protects the body from the outside. Various chemical substances are present in the environment, and the skin is constantly in contact with these chemical substances. For example, numerous chemical substances are present in cosmetics, detergents, toiletries, foodstuffs, pharmaceuticals, and other daily necessities that are indispensable in our lives. As is clear also from the environmental pollution that is getting more and more serious in recent years, many chemical substances are present in environments such as the air, the sea, and the soil. Moreover, the number of chemical substances that exist around us still continues to increase. For example, the number of chemical substances registered in CAS (Chemical Abstracts Service), the world's largest database of chemical substances, reached 100 million in June 2015, and already exceeded 177 million in February 2021. Under these circumstances, it is extremely important to correctly evaluate what effect chemical substances in the environment have on the skin.

Existing methods for evaluating the effects of chemical substances on the skin include methods using mice and guinea pigs, such as GPMT (Guinea Pig Maximization Test) (Non-Patent Literature 1) and LLNA (Local Lymph Node Assay) (Non-Patent Literature 2). However, these methods carried out at the expense of the animal life are not only ethically unfavorable but also relatively time-consuming and inefficient. Various *in vitro* methods have been developed to replace the above methods using animals (Non-Patent Literature 3). But, all of the methods are methods modelling an activation step of an immune response in the skin caused by exogenous chemical substances. It evaluates only a small part of the effects of exogenous chemical substances on the skin.

In recent years, it is gradually getting clear that an aryl hydrocarbon receptor (AhR) and a xenobiotic response element (XRE) are involved in foreign body reaction in the skin against chemical substances present in the environment. For example, Non-Patent Literature 4 suggests that environmental pollutants in the atmosphere trigger a foreign body reaction involving an AhR and a XRE in the skin, and that such a foreign body reaction increases the expression of the neurotrophic factor artemin, causing abnormal elongation of nerves into the epidermis, and that such a abnormal elongation of nerves causes hypersensitivity to itching (allonesis), which in turn causes loss of the skin barrier function and atopic dermatitis. On the other hand, Non-Patent Literature 5 suggests that matrix metalloprotease (MMP) is expressed by an activation of an AhR in the skin, and collagen degradation due to the expression of MMPs leads to skin aging and wrinkle formation. As discussed herein, foreign body reactions in the skin involving an AhR and a XRE cause various skin troubles such as atopic dermatitis and wrinkles.

The aryl hydrocarbon receptor (AhR) is a receptor-type transcription factor found as a receptor for dioxins. AhR is known to bind to a ligand molecule such as 2,3,7,8-tetrachlorodibenzo-p-dioxin (TCDD), which is a type of dioxin, and induces the expression of xenobiotic-metabolizing enzymes. When an AhR is activated by binding to a ligand molecule, it translocates into the cell nucleus, recognizes a specific nucleotide sequence present in the enhancer sequence of the transcriptional regulatory region, and activates the expression of downstream genes. The above-mentioned specific nucleotide sequence is called a xenobiotic response element (XRE) and is known to have a common sequence of 5'-CACGC-3'. Since it was found as a receptor for dioxins, it has been thought that the ligands that activate the aryl hydrocarbon receptor (AhR) are mainly polyaromatic hydrocarbons (PAH) and halogenated aromatic hydrocarbons (HAH). However, in recent years, it has become clearer that the aryl hydrocarbon receptor (AhR) is activated not only by polyaromatic hydrocarbons and halogenated aromatic hydrocarbons but also by chemical substances having a wide variety of structures, and that its activation plays an important role in maintaining homeostasis in the living body (Non-Patent Literature 6).

### Citation List

### Non-Patent Literature

[Non-Patent Literature 1] B. Magnusson, and A.M. Kligman, Journal of Investigative Dermatology 1969, 52: 268-276.

[Non-Patent Literature 2] D. A. Basketter et al., Food and Chemical Toxicology 2002, 40:593-598.
[Non-Patent Literature 3] Sebastian Hoffmann et al., Critical Reviews in Toxicology 2018, 48:344-358.
[Non-Patent Literature 4] Takanori Hidaka et al., Nature Immunology 2017, 18:64-73.
[Non-Patent Literature 5] E. Dupont, J. Gomez, and D. Bilodeau, International Journal of Cosmetic Science 2013, 35:224-232.
[Non-Patent Literature 6] Michael S. Denison and Scott R. Nagy, Annual Review of Pharmacology and Toxicology 2003, 43:309-334.

## Summary of Invention

### Technical Problem

As described above, when a foreign body reaction in the skin is triggered, it causes various skin troubles such as the onset of atopic dermatitis, skin aging, and wrinkle formation, and it has become clearer that activation of an aryl hydrocarbon receptor (AhR), which can be a cause of foreign body reaction in the skin, is induced by a wide variety of chemical substances. However, to the best of the present inventors' knowledge, no consideration has been given to foreign body reactions in the skin involving an AhR and a XRE, when evaluating the safety and effects of various products containing chemical substances such as cosmetics, quasi-drugs, and foodstuffs on the skin. Meanwhile, substances that can inhibit such foreign body reactions and can be safely applied to the human body are not known

The present invention has been made to solve the problems of the prior art as described above. From the viewpoint of animal welfare, an object of the present invention is to provide a method for easily and efficiently evaluating the ability of various test samples to trigger foreign body reactions in the skin without using animals. In another aspect of the present invention, an object of the present invention is to provide a substance having an ability to inhibit a foreign body reaction in the skin, and a method for screening the same.

### Solution to Problem

During accumulation of research effort in order to attain the above object, the present inventors focused on presence of an enhancer sequence comprising an XRE, which is an element regulating expression level of downstream genes in response to a foreign body reaction, in a transcription regulatory region of a gene whose expression is enhanced by a foreign body reaction in which an aryl hydrocarbon receptor (AhR) and a xenobiotic responsive element (XRE) are involved. The present inventors then transformed a keratinocyte by a reporter vector comprising an enhancer sequence comprising a xenobiotic responsive element (XRE) and a reporter sequence coding a reporter protein at downstream of said enhancer sequence, made the resulting transformed keratinocyte in contact with a test sample, and found that an expression level of a reporter protein reflecting a level of an activation of a foreign body reaction in the skin by a test sample could be measured. The present inventors thereby accomplished the present invention.

In other words, the present invention attains the above object by providing a method for evaluating an ability of a test sample to trigger a foreign body reaction in the skin, comprising:

(1) a step of making a solution containing said test sample in contact with a keratinocyte transformed by a reporter vector comprising an enhancer sequence comprising a xenobiotic responsive element (XRE) and a reporter sequence encoding a reporter protein at downstream of said enhancer sequence;
(2) a step of measuring an expression level of said reporter protein in said keratinocyte that is made in contact with said solution containing said test sample; and
(3) a step of evaluating an ability of said test sample to trigger a foreign body reaction in the skin based on the measured expression level of said reporter protein.

In another aspect, the present invention attains the above object by providing a method for screening a substance having an ability to inhibit a foreign body reaction in the skin, comprising:

(1) a step of making a test solution containing a candidate substance and a reference solution without said candidate substance in contact with a keratinocyte transformed by a reporter vector comprising an enhancer sequence comprising a xenobiotic responsive element (XRE) and a reporter sequence encoding a reporter protein at downstream of said enhancer sequence;
(2) a step of measuring an expression level of said reporter protein in said keratinocyte that is made in contact with said test solution and in said keratinocyte that is made in contact with said reference solution, and
(3) a step of evaluating said test candidate as having an ability to inhibit a foreign body reaction in the skin, when said expression level of said reporter protein in said keratinocyte that is made in contact with said test solution is

smaller than said expression level of said reporter protein in said keratinocyte that is made in contact with said reference solution. In a preferred embodiment, said test solution and said reference solution may contain a substance that triggers a foreign body response in the skin.

As described above, activation of a foreign body reaction in the skin is known to enhance production of a neurotrophic factor artemin and matrix metalloprotease 9, causing atopic dermatitis and formation of wrinkles. Therefore, in a preferred embodiment, the above method for screening a substance having an ability to inhibit a foreign body reaction in the skin may be preferably used for screening a substance for preventing and/or treating atopic dermatitis and a substance for preventing and/or improving wrinkles.

The present inventors accumulated further research efforts using the above screening method and found that 5-hydroxy-4-phenyl-2(5*H*)butenolide (hereinafter also referred to as "Fraglide-1"), which is a type of butenolide compound contained in Kozu (a Chinese aromatic vinegar), has an ability to inhibit a foreign body reaction in the skin. As a result of a more detailed study on the ability of 5-hydroxy-4-phenyl-2(5*H*)butenolide to inhibit a foreign body reaction in the skin, it was found that 5-hydroxy-4-phenyl-2(5*H*)butenolide remarkably inhibits an expression of a gene transcribed with involvement of a xenobiotic responsive element (XRE), more specifically, an expression of a neurotrophic factor artemin and matrix metalloprotease 9.

Accordingly, in another aspect, the present invention attains the above object by providing an agent for inhibiting production of matrix metalloprotease 9, comprising 5-hydroxy-4-phenyl-2(5*H*)butenolide and/or its salt as an active ingredient. Matrix metalloproteinase 9 is known to degrade type IV collagens and laminins, which are basement membrane components that make up the extracellular matrix, and elastin, which is a dermal matrix component, and the production of matrix metalloproteinase 9 is thought to be a cause of wrinkles. In other words, the above agent for inhibiting matrix metalloprotease 9, comprising 5-hydroxy-4-phenyl-2(5*H*)-butenolide and/or its salt as an active ingredient may be preferably used for preventing and/or treating wrinkles.

On the other hand, in another aspect, the present invention attains the above object by providing an agent for inhibiting production of artemin, comprising 5-hydroxy-4-phenyl-2(5*H*)butenolide and/or its salt as an active ingredient. As previously mentioned, artemin production in the skin causes abnormal proliferation of nerve cells, and is thought to be a cause of atopic dermatitis. In other words, the above agent for inhibiting production of artemin, comprising 5-hydroxy-4-phenyl-2(5*H*)butenolide and/or its salt as an active ingredient may be preferably used for preventing and/or treating atopic dermatitis.

To date, steroids (e.g., prednisolone, hydrocortisone, etc.) and immunosuppressants (e.g., tacrolimus, pimecrolimus, etc.) are known and commonly used for the treatment of allergic dermatitis including atopic dermatitis. Unfortunately, these agents are aimed at suppressing allergic reactions in the skin and alleviating the symptoms of allergic dermatitis, and do not provide a radical treatment of allergic dermatitis. An agent for inhibiting production of artemin in accordance with one aspect of the present invention is distinct from the above-described conventional therapeutic agents for atopic dermatitis. Because it inhibits production of artemin, which is thought to be a cause of atopic dermatitis, there is an advantage that atopic dermatitis may be completely cured.

## Effects of Invention

According to the evaluation method in accordance with one aspect of the present invention, an ability of a test sample to trigger a foreign body reaction in the skin may be evaluated quantitatively with a simple operation. According to the screening method in accordance with one aspect of the present invention, a substance having an ability to inhibit a foreign body reaction in the skin may be screened quickly with a simple operation. According to the agent in accordance with further one aspect of the present invention, an expression of a gene transcribed with involvement of a xenobiotic responsive element (XRE) may be inhibited and production of matrix metalloprotease 9, which may be a cause of wrinkles, and/or artemin, which may be a cause of atopic dermatitis, may be inhibited.

## Brief Description of Drawings

[FIGURE 1] A schematic illustration showing a structure of a reporter vector comprising a xenobiotic responsive element (XRE) and at is downstream a minimal promoter and a sequence coding a reporter protein.

[Figure 2] A figure showing an appearance of a keratinocyte (XRE-NLuc::HaCaT cell) transformed by a reporter vector comprising a xenobiotic responsive element (XRE) and at is downstream a minimal promoter and a sequence coding a reporter protein.

[Figure 3] A figure showing an expression level of a reporter protein in XRE-NLuc::HaCaT cells which were made in contact with a predetermined concentration of FICZ.

[Figure 4] A figure showing an expression level of artemin mRNA in HaCaT cells that were made in contact with a predetermined concentration of FICZ, which was quantified by PCR method.

[Figure 5] A figure showing an expression level of matrix metalloprotease 9 mRNA in HaCaT cells that were made in contact with a predetermined concentration of FICZ, which was quantified by PCR method.

[Figure 6] A figure showing an expression level of a reporter protein in XRE-NLuc::HaCaT cells that were made in contact with a cell culture medium containing FICZ and either Fraglide-1(FG1) or cinnamaldehyde (CA) at predetermined concentrations.

[Figure 7] A figure showing an expression level of artemin mRNA in HaCaT cells that were made in contact with a cell culture medium containing FICZ and Fraglide-1(FG1) at predetermined concentrations.

[Figure 8] A figure showing an expression level of artemin mRNA in HaCaT cells that were made in contact with a cell culture medium containing FICZ and Fraglide-1(FG1) at predetermined concentrations.

[Figure 9] A figure showing an expression level of matrix metalloprotease 9 (MMP9) mRNA in HaCaT cells that were made in contact with a predetermined concentration of Fraglide-1, which was quantified by PCR method.

[Figure 10] A figure showing an expression level of matrix metalloprotease 2 (MMP2) mRNA in HaCaT cells that were made in contact with a predetermined concentration of Fraglide-1, which was quantified by PCR method.

[Figure 11] A figure showing an expression level of matrix metalloprotease 9 (MMP9) protein in HaCaT cells that were made in contact with a predetermined concentration of Fraglide-1, which was evaluated by gelatin zymography.

[Figure 12] A figure showing an expression level of matrix metalloprotease 9 (MMP9) mRNA in HaCaT cells that were made in contact with either retinoic acid or nicotinic acid amide at predetermined concentrations, quantified by PCR method.

[Figure 13] A figure showing an expression level of matrix metalloprotease 2 (MMP2) mRNA in HaCaT cells that were made in contact with either retinoic acid or nicotinic acid amide at predetermined concentrations, quantified by PCR method.

[Figure 14] A figure showing an expression level of TIMP-1 (Tissue Inhibitor of Matrix Metalloprotease-1) mRNA in HaCaT cells that were made in contact with a predetermined concentration of Fraglide-1, which was quantified by PCR method.

[Figure 15] A figure showing an expression level of TIMP-1 (Tissue Inhibitor of Matrix Metalloprotease-1) mRNA in HaCaT cells that were made in contact with either retinoic acid or nicotinic acid amide at predetermined concentrations, which was quantified by PCR method.

[Figure 16] A figure showing the results of evaluating the cell viability of HaCaT cells treated with a predetermined concentration of Fraglide-1 by WST-1 method.

**Description of Embodiments**

The present invention will be described below in more detail.

<1. A method for evaluating an ability to trigger a foreign body reaction in the skin>

First, the method for evaluating an ability of a test sample to trigger a foreign body reaction in the skin according to one aspect of the present invention will be described.

In the present description, a foreign body reaction in the skin refers to a foreign body reaction in the skin, in which a xenobiotic responsive element (XRE) is involved. When a foreign body reaction, in which a xenobiotic responsive element (XRE) is involved, is triggered, an expression of a gene having a xenobiotic responsive element (XRE) in a transcription regulatory region is upregulated. It is thought that this may be because an aryl hydrocarbon receptor (AhR) recognizes a xenobiotic responsive element (XRE) and selectively upregulate expression of genes that are present at downstream of the xenobiotic responsive element (XRE). A foreign body reaction in the skin may also be referred to as a foreign body reaction in the skin, in which an aryl hydrocarbon receptor (AhR) and a xenobiotic responsive element (XRE) are involved.

On the other hand, the ability to trigger a foreign body reaction refers to a property of triggering the above-described foreign body reaction and it is a concept that encompasses degree of its strength. Having a high ability to trigger a foreign body rection means having a property of activating a foreign body reaction strongly. Meanwhile, having a low ability to trigger a foreign body reaction means a property of activating a foreign body reaction is low. Triggering a foreign body reaction refers to, more specifically, making a gene transcribed with involvement of a xenobiotic responsive element (XRE) expressed and/or enhancing the expression of the gene transcribed with involvement of a xenobiotic responsive element (XRE). When a substance having an ability to trigger a foreign body reaction is in contact with a cell in the skin, it makes a gene transcribed with involvement of a xenobiotic responsive element (XRE) expressed or enhances its expression. To be added, expression of a gene refers to production of a protein coded by the gene. Enhancing expression of a gene refers to increasing production of a protein coded by the gene.

A xenobiotic responsive element (XRE) is an enhancer sequence found as a nucleic acid sequence specifically recognized by an activated aryl hydrocarbon receptor (AhR: aryl hydrocarbon receptor), and is known to have the following nucleic acid sequence: 5'-CACGC-3'. It is known that, when an aryl hydrocarbon receptor (AhR) present in the cytoplasm gets activated by binding to AhR-activating ligand molecules, it translocates to the cell nucleus and specifically enhances expression of genes having an enhancer sequence containing a xenobiotic responsive element (XRE) in their transcription regulatory region.

On the other hand, the skin is an organ that protects the external surface of a living body, being composed of skin cells such as keratinocytes, fibroblasts, and corneocytes. The skin has the three-layered structure consisting of epidermis, dermis and subcutaneous tissue. Foreign body reaction in the skin is thought to be triggered mainly by binding of exogenous foreign substance and aryl hydrocarbon receptors (AhR), which are expressed in keratinocytes that account for about 95% of all epidermal cells. Accordingly, keratinocytes may be preferably used in the evaluation method in accordance with one aspect of the present invention.

Keratinocytes used in the evaluation method according to one aspect of the present invention may be any keratinocytes. For example, keratinocytes derived from animals such as humans, mice, rats, hamsters, rabbits, dogs, cats, pigs, sheep, horses, and cows may be used. However, keratinocytes derived from humans may be preferred. Keratinocytes may be those collected from animals but also may be established cell lines that are commercially available. Examples of established cell lines of human derived-keratinocytes include but not limited to HaCaT cells, NHEK cells, etc.

In the evaluation method in accordance with one aspect of the present invention, keratinocytes as described above are used after transformation. A reporter vector used for transformation may be any reporter vector containing at least an enhancer sequence comprising a xenobiotic responsive element (XRE) and, at its downstream, a sequence encoding a reporter protein. Herein, an enhancer sequence is a nucleic acid sequence that is present in a transcription regulatory region of a gene and has a function of activating expression of the gene in response to a specific stimulus. Sometimes, an enhancer sequence may be simply referred to as an enhancer. Expression of a reporter protein gene that has an enhancer sequence comprising a xenobiotic responsive element (XRE) in its upstream transcription regulatory region may be upregulated by activation of a foreign body rection in the skin. Therefore, the expression of the reporter protein gene in the transformed keratinocytes may reflect the degree of foreign body reaction. Accordingly, the ability of a test sample to trigger a foreign body reaction in the skin may be evaluated by using the keratinocytes transformed by the reporter vector as described above.

As described above, a xenobiotic responsive element (XRE) is a nucleic acid sequence that may be found in a transcription regulatory region of a gene whose expression is upregulated by activation of an aryl hydrocarbon receptor (AhR), and is an enhancer sequence comprising the following nucleic acid sequence as its core sequence: 5'-CACGC-3'. A reporter vector used in the method in accordance with one aspect of the present invention contains at least one xenobiotic responsive element (XRE) in a transcription regulatory region at upstream of a sequence encoding a reporter protein. However, the reporter vector may contain repeating xenobiotic responsive elements (XREs). From the viewpoint of enhancing sensitivity of the method, the reporter vector may preferably contain two or more xenobiotic responsive elements (XREs) and more preferably three or more xenobiotic responsive elements (XREs). As a xenobiotic responsive element, nucleic acid sequences that are found at upstream of a gene such as P4501A1 (CYP1A1) gene, P4501B1 (CYP1B1) gene, glutathione-S-transferase gene, quinone reductase gene, and UDP-glucuronosyltransferase gene may be used. The nucleic acid sequences of these genes may be obtained from public databases such as the database of National Center for Biotechnology Information (NCBI).

On the other hand, the reporter vector used for transformation contains a nucleic acid sequence encoding a reporter protein at downstream of an enhancer sequence comprising a xenobiotic responsive element (XRE). The reporter protein may be any reporter protein as long as a signal reflecting its expression level can be directly or indirectly measured. An appropriate reporter protein may be selected depending on the measurement equipment available to a tester who perform the experiment. For example, when a luminescence photometer is available, a luminescent enzyme protein such as firefly luciferase, Renilla luciferase, marine copepod luciferase, and Oplophorus gracilirostris luciferase may be used as the reporter protein. When a fluorometer or a fluorescence microscope is available, a fluorescent protein such as blue fluorescent protein (BFP), green fluorescent protein (GFP), yellow fluorescent protein (YFP), and red fluorescent protein (RFP) may be used as the reporter protein. When an absorptiometer is available, a chromogenic enzyme protein such as β-galactosidase may be used as the reporter protein. The nucleic acid sequences encoding a reporter protein may be obtained from public databases such as the database of National Center for Biotechnology Information (NCBI).

In addition to the sequences described above, the reporter vector used for transformation may contain various sequences that are necessary for expression of the above-mentioned reporter protein in keratinocytes into which the reporter vector is introduced. For example, the reporter vector used for transformation may contain a promoter sequence, a sequence that confers a ribosome binding site to mRNA, a terminator sequence for transcription termination, an origin of replication sequence, a sequence that encodes a resistance gene that confers resistance to a selection drug, and the like.

The reporter vector used in the evaluation method in accordance with one aspect of the present invention may be those that are commercially available or those that are prepared by oneself. For example, a reporter vector comprising an enhancer sequence comprising a xenobiotic responsive element (XRE) may be obtained from Promega Corporation (For example, the product name "pNL[NLucP/XRE/Hygro]Vector", Catalogue No. CS186808). On the other hand, the reporter vector used in the evaluation method in accordance with one aspect of the present invention may be prepared by introducing an enhancer sequence comprising a xenobiotic responsive element (XRE) in a commercially available reporter vector comprising a multiple cloning site in accordance with the conventional method.

Meanwhile, any type of reporter vector may be used for transformation. Examples of the reporter vector include but not limited to plasmids, cosmids, phagemids, virus vectors, and the like. Any reporter vector that can express in targeted keratinocytes may be appropriately selected.

The transformed keratinocyte may be prepared by introducing the above-described reporter vector to a keratinocyte in accordance with a conventional method in this technical field. The method for introducing the reporter vector to a keratinocyte may not be limited to a specific one. Any appropriate method including physical introduction method or chemical introduction method may be used. Examples of physical introduction method include but not limited to the electroporation method, the sonoporation method, and the microinjection method. Meanwhile, examples of chemical introduction method include but not limited to the calcium phosphate method, the lipofection method using liposomes, and the transfection method using an appropriate delivery means such as cationic lipids, lipidoids, cationic polymers, cell penetrating peptides, antibodies, antibody fragments, proteins, nanoparticles, microparticles, and emulsions.

On the other hand, from the viewpoint of obtaining stable measurement results, the transformed keratinocytes used in the evaluation method in accordance with one aspect of the present invention may be preferably a transformant in which a DNA introduced by the above reporter vector is stably introduced, in other words, a stably transformed keratinocyte. The stably transformed keratinocyte can be established in accordance with a conventional method in this technical field, for example, by introducing a reporter vector comprising a selection drug resistance gene into keratinocytes and culturing the keratinocytes introduced with the reporter vector in a cell culture medium containing the predetermined amount of the selection drug for about 2 weeks. The cells stably introduced with the reporter vector expresses the selection drug resistance gene. Meanwhile, the cells transiently but not stably introduced with the reporter vector show decreased expression of the selection drug resistance gene over time, resulting in the cell death in the cell culture medium containing the selection drug. Therefore, by incubating the keratinocytes for a predetermined period of time in the cell culture medium containing the selection drug, the stably transformed keratinocytes can be obtained. Examples of selection drugs include but not limited to hygromycin B, zeocin™, geneticin™, puromycin, and blasticidin.

On the other hand, a substance used as a test sample in the evaluation method in accordance with one aspect of the present invention may be any kind of substance, including both natural substance and artificially synthesized substance as well as both organic compounds and inorganic compounds. Examples of such a substance include but not limited to low-molecular-weight organic compounds, amino acids, lipids, peptides, proteins, nucleotides, nucleic acids, carbohydrates, natural polymers, synthetic polymers, and combinations thereof. Moreover, the test sample does not necessarily have to be an isolated substance, but also may be a mixture of multiple substances. Examples of such a mixture include but not limited to plant extracts; cosmetics such as lotions, makeup bases, serums, moisturizing creams, milky lotions, blushes, lipsticks, hand creams, lip balms, mascara, eye shadows; daily necessities such as laundry detergents, dishwashing detergents, facial soaps, bath additives including bath salt, bath bomb, bath oil, bubble bath soap, etc., hand soaps, body soaps, shampoos, hand sanitizers; and quasi-drugs such as ointments, insect repellent, medicinal cosmetics, and sunscreen creams. When the test sample is the product as described above, the evaluation method in accordance with one aspect of the present invention is suitably used for safety evaluation and skin compatibility evaluation of the product. The test sample may also be river water, lake water, well water, spring water, tap water, or sea water.

To be added, it is known that production of matrix metalloprotease 9, which may cause wrinkle formation, and artemin, which causes atopic dermatitis, may be activated when a foreign body reaction in the skin, in other words, a foreign body reaction in the skin, in which a xenobiotic responsive element (XRE) is involved, is triggered (For example, Non-Patent Literature 4 and 5). Accordingly, the evaluation method in accordance with one aspect of the present invention may be preferably used as a method for evaluating an ability of a test sample to cause wrinkles and/or an ability to cause atopic dermatitis.

Each step of the evaluation method in accordance of one aspect of the present invention will be described below.

(1) A step of making a transformed keratinocyte in contact with a solution containing a test sample.

This is a step of making a transformed keratinocyte in contact with a solution containing a test sample, which is a target of evaluation. The solution containing the test sample may be prepared by dissolving the test sample by mixing it with an appropriate solvent such as purified water, physiological saline, or cell culture medium. When the test sample is insoluble in water, it may be used by suspending or dispersing it in water, or it may be used by dissolving it

in an organic solvent such as DMSO, which has low cytotoxicity and is miscible with water, and diluting the resulting mixture with a cell culture medium or physiological saline. By adding the thus prepared solution/suspension/dispersion (hereinafter simply referred to as "solution" unless otherwise specified) containing the test sample to cell culture plates or cell culture dishes on which the transformed keratinocytes are present, the solution containing the test samples and the transformed keratinocyte can be in contact with each other. It may be preferable that ingredients other than the test samples in the solution containing the test sample substantially do not cause a foreign body reaction in the skin. For example, a cell culture medium and physiological saline may be preferably used.

From the viewpoint of making the test sample in sufficient contact with the keratinocyte and making the reporter protein expressed in an amount that sufficiently reflects the foreign body reaction triggered by the test sample, it may be preferable that the solution containing test sample and the keratinocyte are incubated for a predetermined period of time after they are made in contact with each other and before they are used in the later-described step (2). For example, after the solution containing test sample and the keratinocyte are made in contact with each other it may be preferable to incubate at least 6 hours or longer, more preferably 12 hours or longer, further preferably 18 hours or longer, and further more preferably 24 hours or longer. Incubation can be performed at 37°C, 5% $CO_2$ condition in accordance with a conventional method.

On the other hand, as a negative control, a solution that is substantially the same as the solution containing the test sample except that it does not contain the test sample (hereinafter also referred to as "a solution without the test sample") may be used. By using the negative control, the ability to trigger a foreign body reaction in the skin of the test sample may be more accurately evaluated. The solution without the test sample may be, for example, a cell culture medium used for preparing the solution containing the test sample. When the negative control is used, a keratinocyte that was made in contact with the solution without the test sample instead of the solution containing the test sample may be used in the below described step (2).

(2) A step of measuring an expression level of a reporter protein

This is a step of measuring an expression level of the reporter protein in the keratinocyte that was made in contact with the solution containing the test sample in the previous step (1), and, when the negative control was used, an expression level of the reporter protein in the keratinocyte that was made in contact with the solution without test sample.

The expression level of the reporter protein may be measured by quantitatively measuring the intensity of signals derived from the reporter protein that reflects the expression level of the reporter protein. The intensity of signals derived from the report protein may be measured by appropriate means depending on the type of reporter proteins. For example, when a luminescent enzyme protein, such as firefly luciferase, Renilla luciferase, marine copepod luciferase, and Oplophorus gracilirostris luciferase is used as the reporter protein, the intensity of signals derived from the reporter protein may be measured by using a substrate that has a property of emitting luminescence subject to enzymatic processing by the luminescent enzyme protein and a luminescence photometer for measuring the intensity of the emitted luminescence. On the other hand, when a fluorescent protein such as blue fluorescent protein (BFP), green fluorescent protein (GFP), yellow fluorescent protein (YFP), and red fluorescent protein (RFP) is used as the reporter protein, the intensity of fluorescence emitted by the fluorescent protein may be measured using a fluorometer. When a chromogenic enzyme protein such as β-galactosidase is used as the reporter protein, the intensity of signals derived from the reporter protein may be measured by using a substrate that has a property of emitting fluorescence or a property of showing absorption at specific wavelength subject to enzymatic processing by the chromogenic enzyme protein and a fluorometer for measuring the fluorescence intensity or an absorptiometer for measuring the absorbance, respectively. When β-galactosidase is used as the reporter protein, examples of substrates include but not limited to 5-bromo-4-chloro-3-indolyl-β-D-galactopyranoside, 2-nitrophenyl-β-D-galactopyranoside, and fluorescein-β-D-galactopyranoside.

(3) A step of evaluating an ability to trigger a foreign body response

Since the expression level of the reporter protein produced in the transformed keratinocyte used in the method in accordance with one aspect of the present invention reflects the degree of a foreign body reaction in which a xenobiotic responsive element (XRE) is involved, the ability to trigger a foreign body reaction can be evaluated based on the expression level of the reporter protein. In other words, it means that the greater the expression level of the reporter protein, the higher the ability to trigger a foreign body reaction. The expression level of the reporter protein is typically evaluated by comparing the expression level of the reporter protein in the keratinocyte that was made in contact with the solution containing the test sample and that in the keratinocyte that was made in contact with the solution without the test sample (the negative control). Namely, if the expression level of the reporter protein in the keratinocyte was made in contact with the solution containing the test sample is higher compared to that in the keratinocyte was made in contact with the solution without the test sample (the negative control), it can be evaluated that the test sample has an ability to trigger a foreign body reaction in the skin. It goes without saying that the greater the degree of increase in the

expression level of the reporter protein, the stronger the ability to trigger a foreign body reaction.

In a preferred embodiment, the evaluation method in accordance with one aspect of the present invention may further contain, before or after the above-described step (2), a step of measuring the number of viable cells of the keratinocyte that was made in contact with the solution containing the test sample, and, when the negative control is used, the number of viable cells of the keratinocyte that was made in contact with the solution without the test sample. When the number of viable cells is measured, the expression level of the reporter protein per viable cell units may be obtained by dividing the expression level of the reporter protein measured in the step (2) by the number of viable cells measured in this step. Since the influence of variations in the number of viable cells between samples can be corrected, it becomes possible to more accurately evaluate the ability to trigger a foreign body reaction. The number of viable cells can be measured according to a conventional method in this technical field. For example, the MTT method using a reductive chromogenic reagent that develops color depending on the activity of mitochondrial dehydrogenase in living cells, as well as the colorimetric methods similar to the MTT method, including the XTT method, the MTS method, the WST method, and the like may be used to measure the number of viable cells.

<2. A method for screening a substance having an ability to inhibit a foreign body reaction in the skin>

On the other hand, a substance that decreases the expression amount of the reporter protein in the above-described transformed keratinocytes can be evaluated as a substance having an ability to inhibit a foreign body reaction in the skin. Therefore, in another aspect, the present invention provides a method for screening a substance having an ability to inhibit a foreign body reaction in the skin. The screening method will be described below.

An ability to inhibit a foreign body reaction means a property of inhibiting a foreign body reaction. Inhibiting a foreign body reaction means decreasing the expression of a gene that is transcribed with involvement of a xenobiotic responsive element (XRE). Herein, as already mentioned, the expression of a gene means that a protein encoded by the gene is produced. Inhibiting the expression of a gene means decreasing a production amount of a protein encoded by the gene.

A candidate substance that is to be screened may be any type of substances. For example, a candidate substance may be a natural substance or an artificially synthesized substance. It may be an organic compound or an inorganic compound. Examples of candidate substances include but not limited to low-molecular-weight organic compounds, amino acids, lipids, peptides, proteins, nucleotides, nucleic acids, saccharides, natural polymers, synthetic polymers, and combinations thereof. A candidate substance may not necessarily be an isolated substance but also may be a mixture of multiple substances. Examples of such a mixture include but not limited to a plant extract.

Examples of a gene that is transcribed with involvement of a xenobiotic responsive element (XRE) include but not limited to a gene encoding matrix metalloprotease 9, which causes wrinkles, and a gene encoding artemin, which causes atopic dermatitis. Accordingly, it can be said that a substance having an ability to inhibit a foreign body reaction in the skin has the property of inhibiting the expression of the above genes. In other words, the screening method according to one aspect of the present invention may be preferably used as a method for screening a substance for preventing and/or improving wrinkles or a substance for preventing and/or treating atopic dermatitis.

In the screening method according to one aspect of the present invention, the same transformed keratinocyte as explained for the above evaluation method may be used. Therefore, as to the transformed keratinocytes and the method for establishing the same, please see the explanation for the above evaluation method.

Each step of the screening method according to one aspect of the present invention will be described below.

(1) A step of making a transformed keratinocyte in contact with a test solution containing a candidate substance or a reference solution not containing the candidate substance

This is a step of bringing a transformed keratinocyte in contact with a test solution containing a candidate substance and, as a control, a reference solution not containing the candidate substance. A test solution containing a candidate substance, a reference solution not containing a candidate substance, and a method for making these solutions and a transformed keratinocyte in contact are the same as described for the solution containing a test substance in the description of the evaluation method.

In a preferred embodiment, a solution containing a candidate substance and a reference solution not containing the candidate substance may contain a substance that triggers a foreign body reaction in the skin. By evaluating the ability to inhibit a foreign body reaction using the expression level of the reporter protein induced by the reference solution containing the substance that triggers a foreign body reaction as a reference, the detection sensitivity may be improved. A substance that triggers a foreign body reaction in the skin may be any substance that increases the expression of a gene transcribed with involvement of a xenobiotic responsive element (XRE) in the skin. Examples of such a substance include but not limited to 6-formylindolo[3,2-b] carbazole (FICZ) (CAS RN: 17922-91-7), indole-3-carbinol (I3C) (CAS RN: 700-06-1), 2-(1'H-indole-3'-carbonyl)-thiazole-4-carboxylic acid methyl ester (ITE) (CAS RN: 448906-42-1), and

indirubin (CAS RN: 479-41-4). Whether a substance is able to trigger a foreign body response in the skin or not may be evaluated, for example, by using the evaluation method according to another aspect of the present invention.

Meanwhile, from a viewpoint of obtaining a result sufficiently reflecting an ability to inhibit a foreign body reaction of a candidate substance by allowing the candidate substance and the keratinocyte in sufficient contact, it may be preferable to proceed to the later described step (2) after incubation for a predetermined period of time after making the keratinocyte in contact with a test solution containing a candidate substance and a reference solution not containing the candidate substance. The incubation time is not particularly limited but, for example, it may be preferable to incubate for at least 6 hours or more, more preferably for 12 hours or more, further preferably for 18 hours or more, and further more preferably for 24 hours or more after making the keratinocyte and the test solution or the reference solution in contact and before proceeding to the subsequent step (2).

(2) A step of measuring the expression of the reporter protein

This is a step of measuring the expression of the reporter protein in the keratinocyte that was made in contact with the test solution containing the candidate substance and the expression of the reporter protein in the keratinocyte that was made in contact with the reference solution not containing the candidate substance. The method for measuring the expression of a reporter protein is as already described in the description of the foregoing section "<1. Method for evaluating an ability to trigger a foreign body reaction in the skin>".

(3) A step of comparing the expression of the reporter protein

This is a step of comparing the expression of the reporter protein in the keratinocytes that were made in contact with the test solution containing the candidate substance and the expression of the reporter protein in the keratinocytes that were made in contact with the reference solution not containing the candidate substance, which were measured in the previous step (2), and evaluating whether a candidate substance has an ability to inhibit a foreign body reaction in the skin based on the comparison. When the expression of the reporter protein in the keratinocytes that were made in contact with the test solution containing the candidate substance is lower than that in the keratinocytes that were made in contact with the reference solution not containing the candidate substance, it can be evaluated that the candidate substance has an ability to inhibit a foreign body reaction in the skin. Herein, the greater degree of decrease in the expression of the reporter protein in the keratinocytes that were made in contact with the test solution containing the candidate substance compared to that in the keratinocytes that were made in contact with the reference solution not containing the candidate substance indicates the stronger ability to inhibit the foreign body reaction in the skin. Therefore, in accordance with the screening method of the present invention, not only the presence or absence of the ability to inhibit a foreign body reaction of the candidate substance can be evaluated, but also the degree of the ability to inhibit a foreign body reaction of the candidate substance can be evaluated.

In a preferred embodiment, the screening method according to the present invention may further comprise a step of measuring the number of viable cells of the keratinocytes that were made in contact with the test solution and that of the keratinocytes that were made in contact with the reference solution before or after the foregoing step (2). When the number of viable cells is measured, the expression of the reporter protein per unit number of viable cells may be obtained by dividing the expression of the reporter protein measured in the foregoing step (2) with the number of viable cells measured in this step. Thereby, the influence of variations in cell number between samples may be corrected, enabling to evaluate an ability to inhibit a foreign body reaction more accurately. As mentioned already, the number of viable cells may be measured in accordance with a conventional method in the art.

<3. An agent for inhibiting a foreign body reaction in the skin>

Next, an agent for inhibiting a foreign body reaction in the skin in accordance with another aspect of the present invention will be described.

As described above, inhibiting a foreign body reaction means reducing an expression of a gene transcribed with involvement of a xenobiotic responsive element (XRE). Accordingly, an agent for inhibiting a foreign body reaction in the skin may be an agent for inhibiting an expression of a gene transcribed with involvement of a xenobiotic responsive element (XRE).

The agent for inhibiting a foreign body reaction in the skin according to one aspect of the present invention comprises 5-hydroxy-4-phenyl-2(5H)butenolide and/or its salt as an active ingredient. 5-hydroxy-4-phenyl-2(5H)butenolide is a butenolide compound discovered in Kozu (a Chinese aromatic vinegar) and is also called Fraglide-1 or 5-hydroxy-4-phenyl-2(5H)furanone. The chemical structure of 5-hydroxy-4-phenyl-2(5H)butenolide is as shown in the below chemical formula (I) (Aydan H. Yatmaz et al., Journal of Oleo Science, 2017, 66:1381-1386).

[Chem. 1]

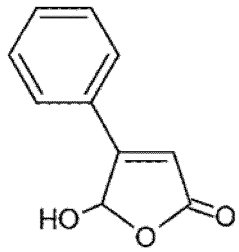

・・・(I)

It is known that 5-hydroxy-4-phenyl-2(5*H*)butenolide shows tautomerism. As a tautomer of 5-hydroxy-4-phenyl-2(5*H*)butenolide, a tautomer represented by the below chemical formula (II) and a tautomer represented by the below chemical formula (III) are known. In the present description, unless otherwise specified, 5-hydroxy-4-phenyl-2(5*H*)butenolide include a tautomer thereof.

[Chem. 2]

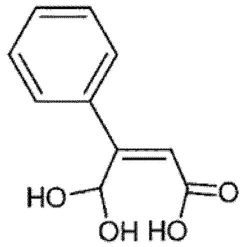

・・・(II)

[Chem. 3]

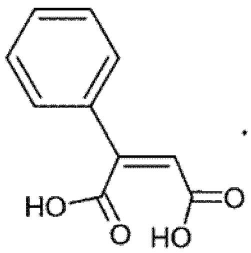

・・・(III)

5-hydroxy-4-phenyl-2(5*H*)butenolide may be obtained by extraction from Kozu (a Chinese aromatic vinegar). A method for obtaining 5-hydroxy-4-phenyl-2(5*H*)butenolide from Kozu is described, for example, in International Publication No. WO2016/006548. Meanwhile, 5-hydroxy-4-phenyl-2(5*H*)butenolide may be also obtained by synthesis. 5-hydroxy-4-phenyl-2(5*H*)butenolide may be synthesized according to the method describe in Japanese Patent Application Publication No. JPH6-211825 and/or Japanese Patent Application Publication No. JPH11-152280.

Meanwhile, a salt of 5-hydroxy-4-phenyl-2(5*H*)butenolide may be any pharmaceutical acceptable salt. 5-hydroxy-4-phenyl-2(5*H*)butenolide and its salt may be in a hydrate form, a solvate form with an organic solvent such as an alcohol, or a anhydrous form. On the other hand, a prodrug which is functionalized with a functional group such that 5-hydroxy-4-phenyl-2(5*H*)butenolide and its salt can be produced in the living body may be also used.

According to findings of the present inventors, 5-hydroxy-4-phenyl-2(5*H*)butenolide and/or its salt show an effect of inhibiting a foreign body reaction in the skin, in other words, an effect of inhibiting an expression of a gene transcribed with involvement of a xenobiotic responsive element (XRE) in the skin. The gene transcribed with involvement of a xenobiotic responsive element (XRE) in the skin has an enhancer sequence comprising a xenobiotic responsive element (XRE) in its transcription regulatory region. As previously mentioned, the expression of such genes is known to be promoted in presence of an activated aryl hydrocarbon receptor (AhR)

Examples of a gene that is transcribed with involvement of a xenobiotic responsive element (XRE) include but not limited to artemin, which is a neurotrophic factor thought to be a cause of atopic dermatitis. As shown in the later described experiment, 5-hydroxy-4-phenyl-2(5*H*)butenolide shows an effect of inhibiting an expression of artemin in a keratinocyte. In other words, an agent comprising 5-hydroxy-4-phenyl-2(5*H*)butenolide as an active ingredient may be used as an agent for inhibiting production of artemin.

Artemin is a member of a glia cell line-derived neurotrophic factor (GDNF) family, and plays a role of inducing proliferation and elongation of neuronal cells via binding to a GDNF family receptor $\alpha$3 (Gfr$\alpha$3). In a mouse in which AhR is constitutively activated, an expression of artemin in the skin can be observed. The expression of artemin in the skin causes an abnormal nerve elongation into the epidermis, evoking hypersensitivity to itching, leading to a loss of skin barrier function and causing atopic dermatitis.

Accordingly, an agent for inhibiting production of artemin, comprising 5-hydroxy-4-phenyl-2(5*H*)butenolide and/or its salt as an active ingredient may be preferably used for preventing and/or treating atopic dermatitis. Herein, preventing and/or treating atopic dermatitis refer to suppressing one or more symptoms of atopic dermatitis or symptoms associated with atopic dermatitis, such as itching, eczema, and lichenification, preventing the onset of these symptoms, or reducing the onset rate of these symptoms.

On the other hand, further examples of a gene that is transcribed with involvement of a xenobiotic responsive element (XRE) include but not limited to matrix metalloprotease 9, which is thought to be a cause of wrinkles. As shown in the later described experiment, 5-hydroxy-4-phenyl-2(5*H*)butenolide shows an effect of inhibiting an expression of matrix metalloprotease 9 in a keratinocyte. In other words, an agent comprising 5-hydroxy-4-phenyl-2(5*H*)butenolide as an active ingredient may be used as an agent for inhibiting production of matrix metalloprotease 9.

Matrix metalloprotease (MMP), including matrix metalloprotease 9, is a generic term referring to metalloproteases (i.e., protease comprising a metal ion at the catalytic site of the active center) that degrade extracellular matrixes. At least 11 kinds of MMPs are known in humans, and they differ in substrate proteins and sites of expression. Matrix metalloprotease 9 (MMP9) is a MMP exhibiting gelatinase activity. MMP9 is closely related to formation of wrinkles, for example, as reported that production of MMP9 is increased in a rat with severe wrinkles formed due to UV irradiation. Accordingly, inhibition of MMP9 expression is thought to be effective in prevention of skin aging and wrinkle formation (Japanese Patent Application Publication No. JP2011-178747; Shinji Inomata et al., Journal of Investigative Dermatology 2003, 120:128-134.)

Therefore, an agent for inhibiting production of matrix metalloprotease 9, comprising 5-hydroxy-4-phenyl-2(5*H*)butenolide and/or its salt as an active ingredient may be preferably used for preventing and/or improving wrinkles. Herein, preventing and/or improving wrinkles refers to preventing formation of wrinkles or improving already formed wrinkles.

Matrix metalloprotease 9 is a factor involved not only in formation of wrinkles but also in many inflammatory skin diseases, such as vitiligo, psoriasis, atopic dermatitis, scleroderma, hypomelanosis and albinism, and is known to be involved in UV-induced skin inflammation and blister formation in pemphigoid (Takashi Kobayashi, Inflammation and Regeneration 2004, 24:578-583.) and depigmentation disorders associated with the above inflammatory skin diseases (Japanese Patent Application Publication (Translation of PCT Application) No. JP2020-504719). Therefore, an agent in accordance with the present invention, in particular, an agent for inhibiting production of matrix metalloprotease-9 in accordance with the present invention may be effectively used for preventing and/or treating various inflammatory skin disease caused by promoted expression of matrix metalloprotease 9 and depigmentation disorders associated with such inflammatory skin diseases. Herein, preventing and/or treating refer to inhibiting these various diseases or one or more symptoms associated with the diseases, or preventing the onset of these diseases, or reducing the onset rate of these diseases, as described above.

Matrix metalloprotease 9, which has a gelatinase activity that degrades the extracellular matrix, such as collagen, gelatin, and elastin, that make up the elastic tissues such as the arteries, the tendons, and the skin, is revealed to be involved not only in inflammatory diseases in the skin but also in various diseases in many organs. For example, matrix metalloprotease 9 is known to play a role in invasion and metastasis of cancer cells (Takashi Kobayashi, Inflammation and Regeneration 2004, 24:578-583), rheumatoid arthritis, osteoporosis, and periodontitis (Japanese Patent Application Publication No. 2015-17048). Therefore, an agent in accordance with the present invention, in particular, an agent for inhibiting production of matrix metalloprotease 9 in accordance with the present invention may be preferably used also for preventing and/or treating various diseases caused by promoted expression of matrix metalloprotease 9, such as cancer, rheumatoid arthritis, osteoporosis, and periodontitis.

On the other hand, as shown in the later described experiment, 5-hydroxy-4-phenyl-2(5*H*)butenolide and/or its salt shows not only an effect of inhibiting production of matrix metalloprotease 9 but also an effect of promoting production of TIMP-1 (Tissue Inhibitor of Matrix Metalloprotease-1), which is an inhibitor of matrix metalloproteases. In other words, in another aspect, the present invention provides an agent for promoting production of TIMP-1, comprising 5-hydroxy-4-phenyl-2(5*H*)butenolide and/or its salts as an active ingredient. As described above, TIMP-1 works as an inhibitor for matrix metalloproteases. Accordingly, an agent for promoting production of TIMP-1, comprising 5-hydroxy-4-phenyl-2(5*H*)butenolide and/or its salt as an active ingredient may be preferably used for preventing and/or improving wrinkles. Needless to say, the above agent for promoting production of TIMP-1 may be preferably used not only for preventing and/or improving wrinkles but also for preventing and/or treating various diseases caused by promoted expression of matrix metalloprotease 9.

There is no particular limitation to the content of 5-hydroxy-4-phenyl-2(5H)butenolide and/or its salt comprised

in the agent in accordance with one aspect of the present invention, but it may be in the range of, for example, 0.0001% by mass to 10% by mass, 0.0005% by mass to 5% by mass, 0.001% by mass to 2% by mass, or 0.005% by mass to 1% by mass.

The agent in accordance with one aspect of the present invention may be preferably used for skin cells, more preferably for skin epidermal cells, and further preferably for keratinocytes. However, it goes without saying that the agent in accordance with one aspect of the present invention may be also applied to other types of cells than the skin cells. For example, the agent in accordance with the present invention may be also applied to cancer cells.

The agent in accordance with one aspect of the present invention may be provided as pharmaceuticals, but it may be provided also as quasi-drugs, cosmetic products, daily necessities, food products, and the like. Herein, examples of food products include but not limited to health foods (functional foods, dietary supplements, health supplements, fortified foods, nutrient adjusted foods, supplements), foods for special dietary uses, and foods with health claims (foods for specified health uses, foods with nutrient function claims). Examples of cosmetic products include but not limited to skincare cosmetics (skin lotion, milky lotion, cream, serum, beauty mask, cleansing, face wash, soap, hand soap, etc.), makeup cosmetics (makeup base, foundation, concealer, face powder, lipstick, blush, eyeshadow, eyeliner, eyebrow, mascara, etc.), body care cosmetics (body soap, body milk, body lotion, body cream, body oil, body powder, sunscreen, sun oil, deodorant spray, bleaching cream, depilatory cream, hand cream, etc.), haircare cosmetics (shampoo, rinse, conditioning shampoo, dry shampoo, hair treatment, conditioner, hair milk, hair cream, hair oil, hair mist, tonic, hair foam, hair gel, wax, pomade, hair liquid, wave agent, hair color, bleach, color rinse, hair restorer, hair growth stimulant, etc.), toothpaste, mouthwash, gargle, bath cosmetics (bath milk, bath salt, bath oil, etc.), and fragrance cosmetics (perfume, cologne, etc.). Daily necessities include but not limited to laundry detergents, kitchen detergents, household detergents, bathroom detergents, disinfectants, insecticide, insect repellents, wet wipes. These quasi-drugs, cosmetics, daily necessities, and food products comprising Fraglide-1 may be preferably used for preventing and/or treating inflammatory skin diseases including atopic dermatitis or preventing and/or improving wrinkles.

The agent in accordance with one aspect of the present invention may be administered by any method. For example, it may be orally administered or parenterally administered. Herein, examples of parenteral administration include but not limited to cutaneous administration, transdermal administration, transmucosal administration, ocular administration, nasal administration, tube administration, inhalation administration, spray administration, subcutaneous injection, intradermal injection, intravenous injection, intramuscular injection, and the like.

The agent in accordance with one aspect of the present invention may be in any formulation form, and an appropriate form may be selected depending on the route of administration. Examples of formulation forms for oral administration include but not limited to tables, capsules, powders, granules, and syrups. Examples of formulation forms for parenteral administration include but not limited to injections, liquids, ointments, poultices, creams, lotions, gels, skin external preparations, sprays, poultices.

The agent according to one aspect of the present invention may further comprise other ingredients depending on its formulation form and/or purpose of use. It may be provided as a composition containing, for example, excipients, base materials, buffering agents, preservatives, chelating agents, antioxidants, surfactants, pearlizers, pH control agents, thickeners and/or one or more of other ingredients formulated in pharmaceuticals, quasi-drugs, cosmetics, daily necessities or food products, etc.

Examples of excipients include but not limited to monosaccharides such as glucose, galactose and fructose; disaccharides such as maltose, trehalose and lactose; oligosaccharides such as maltotriose and maltotetraose; sugar alcohols such as sorbitol, mannitol and maltitol; and polysaccharides such as cellulose (including crystalline cellulose), starch, dextrin, dextran, cyclic sugars (cyclodextrin, isomaltodextrin, etc.).

Examples of base materials include but not limited to water; sea water, deep sea water; lower alcohols such as ethanol and isopropanol; higher alcohols such as cetanol and stearyl alcohol; polyhydric alcohols such as glycerin, butylene glycol, propylene glycol, polyethylene glycol; hydrocarbons such as petroleum jelly and liquid paraffin; silicone oils such as dimethiconol and modified silicone; fats and oils such as coconut oil, olive oil, rice bran oil; and waxes such as jojoba oil and beeswax.

Examples of buffering agents may include but not limited to phosphate buffers, borate buffers, citrate buffers, tartrate buffers, acetate buffers, amino acids, and the like.

Examples of preservatives may include but not limited to benzoic acid; sodium benzoate; 4-hydroxybenzoates such as methyl 4-hydroxybenzoate; quaternary ammonium salts such as benzalkonium chloride; benzyl alcohol; sorbic acid and its salts; thimerosal; methylparaben; ethylparaben; propylparaben; butylparaben; cetylpyridinium chloride, catechin, phenoxyethanol, and the like.

Examples of chelating agents may include but not limited to sodium edetate, disodium edetate, citric acid, phytic acid, gluconic acid, sodium gluconate, etidronic acid, tetrasodium etidronate, pentasodium pentetate, and the like.

Examples of antioxidants include but not limited to sodium bisulfite, sodium sulfite, sodium pyrosulfite, ascorbic acid, ergothioneine, reduced 3,4-dihydroxyphenylethanol elenolic acid, and the like.

Surfactants may be a nonionic surfactant, a cationic surfactant, an anionic surfactant, an amphoteric surfactant,

or a silicone surfactant. Examples of nonionic surfactants include but not limited to polyoxyethylene alkyl ethers such as ceteareth-13 and laureth-7; polyoxyethylene fatty acid esters; polyglycerol esters of fatty acids such as polyglyceryl-2 oleate, polyglyceryl-10 oleate, and polyglyceryl-10 decaisostearate; polyoxyethylene sorbitan fatty acid esters such as polysorbate-20; polyoxyethylene hydrogenated castor oil; sorbitan fatty acid esters such as sorbitan sesquioleate; glycerin fatty acid esters such as glyceryl stearate; and fatty acid alkanolamides such as cocamide DEA, cocamide MEA, cocamide methyl MEA. Examples of cationic surfactants include but not limited to cationized celluloses such as polyquaternium-10; fatty acid amide amine salts; monoalkyl-type quaternary ammonium salts such as behentrimonium methosulfate; and dialkyl-type quaternary ammonium salts. Examples of anionic surfactants include but not limited to fatty acid salts such as laurate, stearate, myristate, and palmitate; alkyl sulfate salts such as sodium lauryl sulfate; alkyl ether sulfate salts such as sodium laureth sulfate; cocoyl alanine salts such as sodium cocoyl alaninate and triethanolamine cocoyl alaninate; cocoyl methyl alanine salts such as sodium cocoyl methyl alanine; lauroyl methyl alanine salts such as sodium lauroyl methylaminopropionate and triethanolamine-lauroyl methylaminopropionate; and cocoyl glutamate. Examples of amphoteric surfactants include but not limited to betaine surfactants such as lauryl betaine. Examples of silicone surfactants include but not limited to dimethicone (methylpolysiloxane), amodimethicone (aminoethylaminopropylmethylsiloxane-dimethylsiloxane copolymer), PEG-10 dimethicone (polyoxyethylene-methylpolysiloxane copolymer).

Examples of pearlizers include but not limited to ethylene glycol distearate and ethylene glycol monostearate.

Examples of pH control agents include but not limited to inorganic acids such as hydrochloric acid and sulfuric acid and salts thereof, organic acids such as lactic acid and succinic acid and salts thereof, inorganic bases such as potassium hydroxide and sodium hydroxide, and organic bases such as triethanolamine.

Examples of thickeners include but not limited to vinyl polymer-type thickeners such as polyvinyl alcohol, polyvinylpyrrolidone, and carbomer (carboxy vinyl polymer); cellulose-type thickeners such as methylcellulose, ethylcellulose, hydroxymethylcellulose, hydroxyethylcellulose, and hydroxypropylmethylcellulose; guar gum; pectin; pullulan; gelatin; carrageenan; alginic acids; other polysaccharides; and the like.

The disclosures of all publications cited in the present description are incorporated herein by reference in their entirety. In addition, when the present description is translated into English and the singular terms such as "a", "an", and "the" are used, these terms include not only single but also plural reference, unless the context clearly dictates otherwise.

Hereinafter, the present invention will be further described with reference to the experimental examples. However, these experimental examples are only illustrative examples of the embodiments of the present invention, and are not intended to limit the scope of the present invention.

<Experiment 1-1: Transformation of Human Epidermal Keratinocytes HaCaT> <XRE-NLuc Vector>

Keratinocytes transformed with a reporter vector comprising an enhancer sequence comprising a xenobiotic responsive element (XRE) and a sequence encoding a reporter protein NLuc (NanoLuc (Registered Trademark)) at downstream of the enhancer sequence were prepared by the following procedure. The structure of the reporter vector (hereinafter also referred to as "XRE-NLuc vector") used for transformation is shown in FIG. 1. As shown in FIG. 1, XRE-NLuc vector used for transformation is a circular plasmid DNA consisting of 4930 base pairs, which has an enhancer sequence in which three xenobiotic responsive elements (XRE) are linked in tandem ("XRE1", "XRE2", and "XRE3" in FIG. 1); has at its downstream a minimal promoter, a nucleotide sequence encoding the reporter protein NLuc ("NLucP reporter" in FIG. 1), and a polyadenylation signal derived from simian virus 40 (SV40) ("SV40 Late poly (A)" in FIG. 1); and at further downstream thereof has SV40 promoter ("SV40 Promoter" in FIG. 1) and a sequence encoding a resistance gene to the selection agent hygromycin B ("Hyg R" in FIG. 1). It also has a polyadenylation signal ("Poly(A) signal" in FIG. 1) at upstream of the enhancer sequence as a sequence for reducing background. In this experiment, a reporter vector (Product Name: "pNL[NLucP/XRE/Hygro]Vector"; Catalogue No.: CS186808) purchased from Promega Corporation was used as a reporter vector having such a structure. However, it goes without saying that a reporter vector prepared by oneself by inserting an XRE sequence and/or a minimal promoter into a reporter vector having a multicloning site may be also used.

<Transformation>

Next, the transformed cell line stably expressing a luciferase reporter which is transcriptionally regulated by a xenobiotic responsive element (XRE) (hereinafter also referred to as "XRE-NLuc::HaCaT") was established by introducing the XRE-NLuc vector to HaCaT cells and treating the cells with the selection agent, hygromycin B, in accordance with the below described procedure. First, HaCaT cells (CLI; Catalogue No.:300493) were seeded at $2\times10^6$ cells/plate in a cell culture plate (Product Name: "TrueLine Cell Culture Dishes"; NIPPON Genetics Co., Ltd.; Catalogue No.: TR4002). Herein, DMEM medium (Dulbecco's Modified Eagle Medium) supplemented with 10% FBS (Fetal Bovine Serum) was used as a cell culture medium. After 7 hours from seeding, the XRE-NLuc vector was introduced into HaCaT cells using

a transfection agent (Product Name: "PEImax"; Polysciences Inc.; Catalogue No.: 24765-1). In detail, 20 μL of 0.1% aqueous solution of PEImax and 10 μL of 500 μg/mL XRE-NLuc vector were mixed, and the total volume of the resulting mixture was added to the cell culture medium. After 48 hours incubation at 5% $CO_2$, 37°C, the DMEM medium was removed, and a new DMEM medium containing 0.08 wt% of hygromycin B (a selection agent) was added. The cell culture was continued in a DMEM medium containing 0.08 wt % of hygromycin B for in total 14 days while changing the cell culture medium about every 4 days. Thereby, a XRE-NLuc::HaCaT cell line stably expressing XRE-NLuc was obtained. A photograph of the obtained XRE-NLuc::HaCaT cells taken with an optical microscope is shown in FIG. 2. As seen in FIG. 2, the morphology of XRE-NLuc::HaCaT cells was unchanged from HaCaT cells before transformation.

<Experiment 1-2 Evaluation of the ability to trigger a foreign body reaction using XRE-NLuc::HaCaT cells>

Using XRE-NLuc::HaCaT cells prepared in Experiment 1-1, the ability of test samples to trigger a foreign body reaction in the skin was evaluated. The detailed procedure is described below.

<Contact with test samples>

First, XRE-NLuc::HaCaT cells were suspended in a DMEM medium at the density of $1 \times 10^5$ cell/mL, and 100 μL of the thus obtained suspension was added to each well of white 96 well plates (Thermo Fisher Scientific, MA, U.S.A.; Catalogue No.: 136102) so that the number of the cells in each well was 10,000 cells/well. In accordance with a conventional method, the cells were incubated at 37°C, 5% $CO_2$ for 24 hours. Thereafter, the DMEM medium was removed, and 100 μL of a new DMEM medium containing FICZ (6-Formylindolo[3,2-b]carbazole; CAS RN: 172922-91-7) as a test sample at a predetermined concentration was added, followed by further incubation for 24 hours at 37°C, 5% $CO_2$. FICZ is known as a substance that triggers a foreign body reaction in which XRE is involved. Meanwhile, negative controls were prepared by adding 100 μL of a DMEM medium containing no FICZ instead of the DMEM medium containing FICZ, which were also incubated for 24 hours at 37°C, 5% $CO_2$.

<Measurement of Viable Cell Count>

After the above-described incubation for 24 hours, the number of cells in each well was counted by a colorimetric method based on the degradation of tetrazolium salt (WST-1) by mitochondrial dehydrogenase in viable cells. Specifically, 3 μL of Premix WST-1 (Product Name "Premix WST-1 Cell Proliferation Assay System"; Takara Bio Inc., Shiga, Japan; Catalogue No.: MK400) was added to each well of 96 well plates, and then the cells were cultured for 1 hour at 37°C, 5% $CO_2$. Thereafter, 93 μL of cell culture supernatant was removed from each well to measure the absorbance of the supernatant in each well. The removed supernatants were transferred to transparent 96 well plates, and then the absorbance at 450 nm (A450) and, as a reference wavelength, the absorbance at 670 nm (A670) were measured using iMark microplate reader (Bio-Rad; Catalogue No.:168-1130JA). A value (A450-A670) was obtained by subtracting the absorbance at 670 nm (A670) from the absorbance at 450 nm (A450). The relative viable cell count (%) was expressed as a relative value with the value (A450-A670) obtained for the negative control as 100%.

<Measurement of Reporter Protein Expression>

Meanwhile, the expression level of the reporter protein was measured using samples left on the white 96-well plates after removing the supernatants for the viable cell count measurement. Briefly, 7 μL of Nano-Glo Luciferase Assay System (Promega; Catalogue No.: N110) was added to each well of the white 96-well plate. After 3 mins, the luminescence intensity obtained from the reporter protein produced by XRE-NLuc::HaCaT cells in each well was measured using GloMax Navigator Microplate Luminometer (Promega; Catalogue No.: GM2000). The obtained luminescence intensities were divided by the relative viable cell counts obtained by the above-described colorimetric method to obtain the expression level of the reporter protein (luminescence intensity) per unit number of viable cells.

The obtained results were shown in FIG. 3. In FIG. 3, the luminescence intensity per unit number of viable cells obtained from XRE-NLuc::HaCaT cells incubated with each of the DMEM medium containing FICZ at different concentrations was shown as a relative value, with the luminescence intensity per unit number of viable cells obtained for the negative control set to 1. The results shown in FIG. 3 were the mean obtained from four independent experiments, and the error bars represent the standard error (SEM). In the figure, "*" and "**" indicate statistically significant differences ($p<0.05$ and $p<0.01$, respectively) from the negative control ("FICZ Concentration 0 nM" in FIG. 3).

As shown in FIG. 3, the luminescence intensity from XRE-NLuc::HaCaT cells was increased along with the concentration of FICZ, which activates the xenobiotic responsive element (XRE). This result indicates that the expression level of the reporter protein produced by XRE-NLuc::HaCaT cells reflects the degree of activation of foreign body reaction in which the xenobiotic responsive element (XRE) is involved. In other words, the ability of a test sample to trigger a

foreign body reaction in the skin can be evaluated by monitoring the expression level of the reporter protein, that is the luminescence intensity in this experiment, produced by XRE-NLuc::HaCaT cells, which is a keratinocyte transformed by XRE-NLuc vector; i.e., a reporter vector comprising an enhancer sequence comprising a xenobiotic responsive element (XRE) and a reporter sequence encoding a reporter protein at downstream of the enhancer sequence. Of note, XRE-NLuc::HaCaT cells showed about three times higher luminescence intensity than the negative control without FICZ, even when they were cultured in the medium containing very low concentration of FICZ of only 4 nM. This result indicates that the ability to trigger a foreign body reaction in the skin can be evaluated with very high sensitivity in accordance with the method of the present invention.

<Experiment 1-3 Evaluation of the ability to trigger a foreign body reaction in the skin by PCR method>

Next, the ability to trigger a foreign body reaction in the skin was evaluated by measuring change in the expression level of mRNA caused by a foreign body reaction in HaCaT cells by a PCR method, in order to compare the sensitivity of the evaluation method of the present invention using XRE-NLuc::HaCaT cells with that of the conventional evaluation method. In the following experiment, artemin and matrix metalloprotease 9 were selected as target genes. It is known that the expression of these genes is activated by foreign body reaction in the skin in which xenobiotic responsive element (XRE) is involved. The detailed procedure for the experiment is described below:

<Contact with test samples>

First, HaCaT cells (CLI; Catalogue No.: 300493) were suspended in a DMEM medium at the density of $1 \times 10^5$ cell/mL, and 100 μL of the thus obtained suspension was added to each well of white 96 well plates (Thermo Fisher Scientific, MA, U.S.A.; Catalogue No.: 136102) so that the number of the cells in each well was 10,000 cells/well. In accordance with a conventional method, the cells were incubated at 37°C for 24 hours in the presence of 5% $CO_2$. Thereafter, the DMEM medium was removed, and 100 μL of a new DMEM medium containing FICZ at a predetermined concentration (i.e., 5 nM, 50 nM, 500 nM, and 5,000 nM) was added, followed by further incubation for 24 hours at 37°C and 5% $CO_2$. Meanwhile, negative controls were prepared by adding 100 μL of a DMEM medium containing no FICZ instead of the DMEM medium containing FICZ, which were also incubated for 24 hours at 37°C and 5% $CO_2$.

<Extraction of RNA and preparation of cDNA>

After the above-described incubation for 24 hours, total RNA was extracted and cDNA was prepared using a PCR kit "SuperPrep II Cell Lysis & RT Kit for qPCR" (Catalogue No.: SCQ-401) manufactured by Toyobo Co., Ltd. (Osaka, Japan) in accordance with a method described in the manual attached to the kit. Briefly, after the above-described incubation for 24 hours, cell culture medium was removed from each well and the cells were washed with PBS (Phosphate-buffered saline). Thereafter, each well was added with 10 μL of a lysis reagent, which was prepared by mixing 58.7 μL of "Lysis solution", 0.3 μL of "gDNA Remover" and 1 μL of "RNase Inhibitor" attached to the above PCR kit. The cell culture plate was shaken for about 30 seconds, and allowed to stand at room temperature for 5 minutes, to obtain a cell lysate containing total RNA that can be used as a template for reverse transcription reaction. Herein, "Lysis Solution" was a cell lysis agent to lyse cells; "gDNA remover" was a reagent containing DNase I that degrades genome DNA; and "RNase Inhibitor" was a reagent to inhibit degradation of RNA by cellular components such as RNase, etc.

Next, cDNA was synthesized from the extracted RNA by reverse transcription. Firstly, a master mix for reverse transcription reaction was prepared by mixing 8 μL of "5X RT Master Mix" and 24 μL of "Nuclease-free water", which were attached to the above PCR kit "SuperPrep II Cell Lysis & RT Kit for qPCR" manufactured by Toyobo Co., Ltd. (Osaka, Japan). Preparation of the master mix for reverse transcription reaction was carried out on ice. In a PCR tube, 32 μL of the obtained master mix was dispensed and then 8 μL of the above cell lysate present in each well was added to make a total volume 40 μL, which was used for the following reverse transcription reaction. The reverse transcription reaction was carried out under the conditions of 37°C for 15mins, 50°C for 5 mins, and 98°C for 5mins. The thus obtained cDNA solution was added with 80 μL of sterile water and then frozen at -20 °C for storage.

Quantitative PCR was carried out by using THUNDERBIRD Next SYBR qPCR Mix (Toyobo Co., Ltd., Osaka, Japan; Catalogue No. QPX-201) and StepOne Real-Time PCR System (Thermo Fisher Scientific, MA, USA). Specifically, a mixed solution was prepared by mixing 6 pmole of forward primer and reverse primer for a target gene having a nucleotide sequence shown in below Table 1 with 5 μL of a solution obtained by diluting THUNDERBIRD Next SYBR qPCR Mix, which was provided as a 2x concentrated premix reagent, with sterile water to 1x concentration. On the other hand, after defrosting the frozen cDNA solution, the defrosted solution was diluted 5 times with sterile water to obtain the diluted cDNA solution. By mixing 5 μL of the obtained cDNA solution and 5 μL of the mixed solution, 10 μL of a reaction solution for PCR was obtained. The reaction solution was used for quantitative PCR. Based on Ct (cycle threshold) value obtained by quantitative PCR, an amount of mRNA of each target gene was obtained. Herein, an amount

of mRNA of GAPDH, an endogenous gene, was used as a reference. An amount of mRNA of each target gene was divided by an amount of mRNA of GAPDH, and thereby normalized.

[Table 1]

| Gene | | Nucleotide Sequence | |
|---|---|---|---|
| GAPDH | Forward primer | 5'-AGCCACATCGCTCAGACAC-3' | (SEQ ID. NO. 1) |
| | Reverse primer | 5'-GCCCATACGACCAATCC-3' | (SEQ ID. NO. 2) |
| Artemin | Forward primer | 5'-CGTCCTGGTGTTGARAGAGA-3' | (SEQ ID. NO. 3) |
| | Reverse primer | 5'GCGGAGAGACCAAGTGGA-3' | (SEQ ID. NO. 4) |
| MMP9 | Forward primer | 5'-GAACCAATCTCACCGACAGG-3' | (SEQ ID. NO. 5) |
| | Reverse primer | 5'-GCCACCCGAGTGTAACCATA-3' | (SEQ ID. NO. 6) |

The condition for quantitative PCR was as shown in Table 2. The above reaction solution for PCR was maintained at 95°C for 20 sec, and then subjected to 40 cycles of denaturation-annealing-extension reaction, each cycle consisting of a denaturation step at 95°C for 3 sec and an annealing-extension step at 60°C for 30 sec. After 40 cycles, the reaction solution was maintained at 95°C for 15 sec, then at 60°C for 60 sec, and then at 95°C for 15 sec.

[Table 2]

| Step | Temperature | Time | Number of Cycles |
|---|---|---|---|
| 1 | 95°C | 20 sec | 1 |
| 2 | 95°C | 3 sec | 40 |
| | 60°C | 30 sec | |
| 3 | 95°C | 15 sec | 1 |
| 4 | 60°C | 60 sec | 1 |
| 5 | 95°C | 15 sec | 1 |

The obtained results were shown in FIG. 4 and FIG. 5. In FIG. 4 and FIG. 5, the mRNA expression levels in HaCaT cells incubated in DMEM mediums containing different concentrations of FICZ were shown in relative values, with the mRNA expression level in HaCaT cells incubated in DMEM medium containing no FICZ obtained as negative control set to 1. The results shown in FIG. 4 and FIG. 5 were the mean obtained from four and three independent experiments, respectively. The error bars represent the standard deviation (SD).

As shown in FIG. 4, the expression level of artemin mRNA in HaCaT cells was increased along with the concentration of FICZ added to the cell culture medium. This result indicates that the activation of the foreign body reaction in HaCaT cells can also be detected by quantification of mRNA by the PCR method. However, no change was observed in the expression level of artemin mRNA, when the concentration of FICZ was 50 nM or lower. A high concentration of FICZ of at least 500 nM, more preferably of as high as 5,000 nM, was necessary to observe a significant increase in the expression level of artemin mRNA.

Meanwhile, as shown in FIG. 5, the expression level of MMP9 mRNA in HaCaT cells was increased along with the concentration of FICZ added to the cell culture medium, similarly as observed in artemin. However, the degree of increase in the expression level was still very small. In order to observe a significant increase in the expression level of MMP9 mRNA, a high concentration of FICZ of about 5000 nM was necessary.

In contrast, as shown in Experiment 1-2, by the evaluation method in accordance with one embodiment of the present invention using XRE-NLuc::HaCaT cells, a strong luminescence intensity that was about three times higher than that of the negative control to which FICZ was not added was observed, even when FICZ was used at a very low concentration of only 4 nM. This result indicates that, in accordance with the evaluation method of the present invention, an ability of each test sample to trigger a foreign body reaction in the skin can be evaluated much more quickly with much higher sensitivity compared to the conventional evaluation method, which involves quantification of the expression level of mRNA by the PCR method.

<Experiment 1-4 Evaluation of an ability to trigger a foreign body reaction using XRE-NLuc::HaCaT Cells>

The ability of each test sample to trigger a foreign body reaction was evaluated using XRE-NLuc::HaCaT cells

in the same manner as in Experiment 1-2, except that I3C (Indole-3-carbinol, CAS RN: 700-06-1), ITE (2-(1'H-indole-3'-carbonyl)-thiazole-4-carboxylic acid methyl ester, CAS RN: 448906-42-1), and Indirubin (CAS RN: 479-41-4) were used instead of FICZ as test samples and the amount of each test sample added to the DMEM medium was appropriately changed. From the evaluation results, the maximum effective concentration (the concentration of a test sample at which the expression level of the reporter protein was maximized), EC50 (50% effective concentration), and LC50 (median lethal concentration) were obtained in accordance with a conventional method. The obtained results were shown in Table 3.

[Table 3]

| | | Literature Value | Present Experiment | | |
|---|---|---|---|---|---|
| Test Sample | CAS RN | Maximum Effective concentration Max. Induction (μM) | Maximum Effective concentration Max. Induction (μM) | EC50 (μM) | LC50 (μM) |
| FICZ | 17922-91-7 | 1 ** | 0.5 | 0.012 | >5 |
| I3C | 700-06-1 | - | 350 | 11 | >700 |
| ITE | 448906-42-1 | 10** | 25 | 1.3 | >100 |
| Indirubin | 479-41-4 | 5-10* | 0.25 | 0.006 | >40 |
| * Aneta Novotna et al., Environ. Sci. Technol. 2011, 45, 23, 10133-10139.<br>** Hideo Satsu et al., Cytotechnology 2015, 67, 621-632. | | | | | |

As shown in Table 3, XRE-NLuc::HaCaT cells were responsive to various types of chemical substances. In other words, in accordance with the evaluation method of the present invention, it would be possible to evaluate the ability to trigger a foreign body reaction in the skin of test samples containing a chemical substance with a wide variety of chemical structures. In addition, in accordance with the evaluation method of the present invention, it would be possible not only to evaluate whether individual test samples have the property of triggering a foreign body reaction in the skin, but also to quantitatively evaluate the strength of the ability to trigger a foreign body reaction in the skin as shown in Table 3, because the expression level of the reporter protein by XRE-NLuc::HaCaT cells reflects the degree of the foreign body reaction, in which XRE is involved.

Meanwhile, in Table 3, literature values of the maximum effective concentration of each test sample, in other words, the concentration of the test sample at which a foreign body reaction reaches plateau, were also shown. Although all these literature values are the maximum effective concentrations obtained by the experiment using human liver cancer-derived cell line HepG2 cells but not keratinocytes, when comparing these literature values with the maximum effective concentrations in keratinocytes obtained in this experiment, it was observed that the degree of foreign body reactions activated in keratinocytes and that in human liver cancer-derived cells were markedly different. For example, while the maximum effective concentration of Indirubin in human liver cancer-derived cells was 5-10 μM, the maximum effective concentration of Indirubin in keratinocytes was 0.25 μM, which was less than 1/20 of the maximum effective concentration in human liver cancer-derived cells. This result suggests that Indirubin may trigger a foreign body reaction in keratinocytes at an amount lower than that needed in human liver cancer-derived cells. This result indicates that the evaluation method of the present invention may be very useful for evaluating safety and compatibility of test samples to the skin, because the ability of test sample to trigger a foreign body response in the skin may be appropriately evaluated by the evaluation method of the present invention using keratinocytes.

<Experiment 2: Screening of a substance having a property of inhibiting foreign body reaction in the skin>

As shown in Experiment 1-2, XRE-NLuc::HaCaT cells expressed the reporter protein in an amount corresponding to the degree of the activation of foreign body reaction in which a xenobiotic responsive element (XRE) is involved. The present inventors conceived an idea opposite to Experiment 1-2 that a substance that have a property of inhibiting a foreign body response in which a xenobiotic responsive element (XRE) is involved may be screened using XRE-NLuc::HaCaT cells, and examined the idea. The experimental procedure is described below.

First, in the same manner as in Experiment 1-2, XRE-NLuc::HaCaT cells were suspended in a DMEM medium at the density of $1 \times 10^5$ cell/mL, and 100 μL of the thus obtained suspension was added to each well of white 96-well plates (Thermo Fisher Scientific, MA, U.S.A.; Catalogue No.: 136102) so that a number of the cells in each well was 10,000 cells/well. In accordance with the conventional method, the cells were incubated for 24 hours at 37°C and 5% $CO_2$. Thereafter, the DMEM medium was removed, and 100 μL of a new DMEM medium containing 5 nM of FICZ, which

was confirmed to activate the expression of the reporter protein in Experiment 1-2, and a candidate substance at a predetermined concentration. Meanwhile, reference samples were also prepared by adding 100 μL of a DMEM medium containing 5,000 nM of FICZ but not containing candidate substances. In this experiment, 5-hydroxy-4-phenyl-2(5H)butenolide (hereinafter also referred to as "Fraglide-1"or "FG1") and, as a positive control, cinnamaldehyde, which is known to have an effect of inhibiting a foreign body reaction in which a xenobiotic responsive element (XRE) is involved, were used as test substances. After incubation for 24 hours at 37°C and 5% $CO_2$, the expression level of the reporter protein (luminescence intensity) and the viable cell count were measured in accordance with the method described in Experiment 1-2, and the expression of the reporter protein per unit number of viable cells was obtained. The inhibition rate (Inhibition (%)) of the expression of the reporter protein exerted by each candidate substance was calculated in accordance with the following formula.

[Formula 1]

$$\text{Inhibition (\%)} = \frac{(\text{Reporter Expression})_{\text{FICZ}} - (\text{Reporter Expression})_{\text{FICZ+TEST SAMPLE}}}{(\text{Reporter Expression})_{\text{FICZ}}} \times 100$$

In the above formula, $(\text{Reporter Expression})_{\text{FICZ}}$ indicates the expression of the reporter protein per unit number of viable cells obtained from the reference samples: i.e., the cells incubated in the medium containing 5,000 nM of FICZ but not containing candidate substances. Meanwhile, $(\text{Reporter Expression})_{\text{FICZ+TEST SAMPLE}}$ indicates the expression of the reporter protein per unit number of viable cells obtained from the cells incubated in the medium containing 5,000 nM of FICZ and a candidate substance. The obtained results were shown in FIG. 6. The results shown in FIG. 6 were the mean obtained from three independent experiments. The error bars represent the standard error (SE).

As shown in FIG. 6, the expression of the reporter protein triggered by FICZ (5,000 nM) was inhibited for 8%, 14%, and 16% in XRE-NLuc::HaCaT cells incubated with 12.5 μM, 25 μM and 50 μM of the positive control, cinnamaldehyde, respectively ("CA" in FIG. 6). This result indicates that the foreign body reaction triggered by FICZ was inhibited by addition of cinnamaldehyde. This result further indicates that a substance having a property of inhibiting a foreign body reaction may be obtained by adding a candidate substance that is to be screened together with a substance that triggers the foreign body reaction ("FICZ" in this experiment) to XRE-NLuc::HaCaT cells and by selecting a candidate substance that reduces the expression of the reporter protein compared to when a reference sample that does not contain the candidate substance is applied.

Surprisingly, the foreign body reaction triggered by FICZ (5,000 nM) was inhibited for 58%, 68%, and 83% in XRE-NLuc::HaCaT cells incubated with 12.5 μM, 25 μM and 50 μM of Fraglide-1, respectively ("FG1" in FIG. 6). This result indicates that Fraglide-1 has a property of inhibiting foreign body reaction in which a xenobiotic responsive element (XRE) is involved, and that this inhibitory activity of Fraglide-1 is much superior to that of cinnamaldehyde.

<Experiment 3-1 Inhibition of artemin production by Fraglide-1>

The above experiments showed that Fraglide-1 have a property of markedly inhibiting a foreign body reaction. In order to investigate in more detail into the effect of inhibiting a foreign body reaction shown by Fraglide-1, an effect of Fraglide-1 on the expression level of artemin, which is known to be a gene transcriptionally regulated with involvement of a xenobiotic responsive element, in HaCaT keratinocytes was examined. Specifically, HaCaT cells were made in contact with FCTZ, which is an activator of foreign body reaction, and Fraglide-1, and thereafter changes in the expression level of artemin mRNA by Fraglide-1 were evaluated using quantitative PCR. The procedure is described below.

First, HaCaT cells (CLI; Catalogue No.:300493) were suspended in a DMEM medium at the density of $1 \times 10^5$ cell/mL, and 100 μL of the thus obtained suspension was added to each well of white 96-well plates (Thermo Fisher Scientific, MA, U.S.A.; Catalogue No.: 136102) so that the number of the cells in each well was 10,000 cells/well. In accordance with the conventional method, the cells were incubated for 24 hours at 37°C and 5% $CO_2$. Thereafter, the DMEM medium was removed, and 100 μL of a new DMEM medium containing both FICZ at predetermined concentration (500 nM or 5,000 nM) and Fraglide-1 at a predetermined concentration (0 nM or 250 nM). After incubation for 24 hours at 37°C and 5% $CO_2$, the total RNA was extracted, cDNA was prepared, and quantitative PCR was carrier out using the obtained cDNA in the same manner as described in Experiment 2. The obtained results were shown in FIG. 7. In FIG. 7, the results obtained for each sample were shown as a relative value, with the expression level of artemin mRNA in HaCaT cells incubated 24 hours in DMEM medium containing neither FICZ nor Fraglide-1 set to 1.

As shown in FIG. 7, the expression level of artemin mRNA was enhanced about 3.0-fold and 3.7-fold in the HaCaT cells incubated in the medium containing 500 nM and 5,000 nM of FICZ, respectively ("FG1 -" in FIG. 7) compared to the HaCaT cells incubated in the medium without FICZ. This may be presumably because FICZ activated a foreign body reaction in which XRE is involved. In contrast, no increase in the expression level of artemin mRNA was observed

in the HaCaT cells incubated in the medium containing 250 μM of Fraglide-1 in addition to 500 nM or 5,000 nM of FICZ ("FG1 +" in FIG. 7) compared to the HaCaT cells incubated in the medium without FICZ. This result indicates that the expression of artemin triggered by a foreign body reaction was inhibited by Fraglide-1, presumably because Fraglide-1 inhibited activation of a foreign body reaction by FICZ. In addition, when the cells were treated with Fraglide-1, the expression level of artemin mRNA remained at the similar level as in the HaCaT cells incubated in the medium without FICZ, and importantly no excessive decrease of the artemin expression was observed. It is concluded that Fraglide-1 has an activity of inhibiting abnormal artemin expression by inhibiting a foreign body reaction in the skin, in other words, an activity of normalizing expression of artemin.

<Experiment 3-2: Inhibition of Artemin production by Fraglide-1 - part 2>

As described above, Fraglide-1 inhibited production of artemin induced by FICZ. Then, the following experiment was conducted in order to quantitatively evaluated the effect of inhibiting artemin production exerted by Fraglide-1. Briefly, HaCaT cells were treated with FICZ and Fraglide-1 in the same manner as in Experiment 3-1 except that the concentration of FICZ was set to 5,000 nM and the concentration of Fraglide-1 was set to 0 μM, 0.01 μM, 0.1 μM, 1 μM, 10 μM, and 100 μM, and then the total RNA was extracted, cDNA was prepared, and quantitative PCR was carried out. Based on the obtained result, the inhibition rate (Inhibition (%)) of the artemin mRNA expression triggered by FICZ when a given concentration of Fraglide-1 was added to the cell culture medium together with 5,000 nM of FICZ was calculated in accordance with the following formula.

[Formula 2]

$$\text{Inhibition (\%)} = \frac{(\text{mRNA Expression})_{\text{FICZ}} - (\text{mRNA Expression})_{\text{FICZ+TEST SAMPLE}}}{(\text{mRNA Expression})_{\text{FICZ}} - (\text{mRNA Expression})_{\text{CONTROL}}} \times 100$$

In the above formula, $(\text{mRNA Expression})_{\text{CONTROL}}$ indicates the artemin mRNA expression in the HaCaT cells incubated in the medium containing neither FICZ nor Fraglide-1 (i.e., the non-treated HaCaT cells). $(\text{mRNA Expression})_{\text{FICZ}}$ indicates the artemin mRNA expression in the HaCaT cells incubated in the medium containing 5,000 nM of FICZ. And, $(\text{mRNA Expression})_{\text{FICZ+TEST SAMPLE}}$ indicates the artemin mRNA expression in the HaCaT cells incubated in the medium containing 5,000 nM of FICZ and a given concentration of Fraglide-1.

As shown in FIG. 8, Fraglide-1 inhibited the FICZ-induced expression of artemin in the concentration dependent manner. Surprisingly, a small amount of Fraglide-1 of only 100 nM (0.1 μM) inhibited the expression of artemin triggered by as much as 5,000 nM (5μM) of FICZ by approximately 50%. This result indicates that Fraglide-1 very effectively inhibits the foreign body reaction in which XRE is involved, and normalizes the artemin expression. As discussed above, induction of artemin expression was thought to be a cause of atopic dermatitis, and it has been reported that artemin expression is induced also by chemicals contained in air pollutants (For example, Non-Patent Literature 4). Fraglide-1, which very effectively inhibits artemin expression in the skin, may be very advantageous for use in preventing and/or treating atopic dermatitis.

<Experiment 4-1: Inhibition of Matrix Metalloprotease 9 production by Fraglide-1>

Next, the effect of Fraglide-1 on the expression of matrix metalloprotease 9 (MMP9), which is known as one of other proteins transcriptionally regulated with involvement of a xenobiotic responsive element, in HaCaT cells was examined.

In this experiment, the effect of Fraglide-1 on the expression of MMP9 in the level of mRNA was examined first. Specifically, HaCaT cells were treated with Fraglide-1 in the same manner as described in Experiment 3-1 except that HaCaT cells were incubated for 24 hours in the medium containing 0 μM, 1 μM, 10μM, and 100 μM of Fraglide-1 instead of being incubated for 24 hours in the medium containing both FICZ and Fraglide-1. The total RNA was then extracted, cDNA was prepared, and the quantitative PCR was performed using the obtained cDNA. Herein, the procedure of the quantitative PCR was the same as described in Experiment 3-1 except that the primers shown in below Table 4 were used. In this experiment, the expression of MMP2 mRNA was also quantified in addition to MMP9 mRNA. MMP2 is known as a MMP which is not transcriptionally regulated by a xenobiotic responsive element (XRE).

[Table 4]

| Gene | | Nucleotide Sequence | |
|---|---|---|---|
| GAPDH | Forward primer | 5'AGCCACATCGCTCAGACAC-3' | (SEQ ID. NO. 1) |
| | Reverse primer | 5'-GCCCAATACGACCAAATCC-3' | (SEQ ID. NO. 2) |
| MMP9 | Forward primer | 5'-GAACCAATCTCACCGACAGG-3' | (SEQ ID. NO. 5) |
| | Reverse primer | 5'-GCCACCCGAGTGTAACCATA-3' | (SEQ ID. NO. 6) |
| MMP2 | Forward primer | 5'AGCGAGTGGATGCCGCCTTTAA-3' | (SEQ ID. NO. 7) |
| | Reverse primer | 5'-CATTCCAGGCATCTGCGATGAG-3' | (SEQ ID. NO. 8) |

The obtained results were shown in FIG. 9 and FIG. 10. In FIG. 9 and FIG. 10, the results obtained for each sample were shown as a relative value, with the mRNA expression level of MMP9 or MMP2 in the HaCaT cells incubated for 24 hours in DMEM medium without FICZ (i.e., the non-treated HaCaT cells) set to 1. The results shown in FIG. 9 and FIG. 10 were the mean obtained from three independent experiments. The error bars represent the standard deviation (SD).

As shown in FIG. 9, the expression level of MMP9 mRNA were decreased by approximately 42%, 52%, and 80% in the HaCaT cells incubated in the medium containing 1 μM, 10 μM and 100 μM of Fraglide-1, respectively, compared to when HaCaT cells were incubated in the medium without Fraglide-1. This result indicates that Fraglide-1 has an activity of decreasing the expression of MMP9, which is a MMP whose transcription is regulated by a xenobiotic responsive element (XRE). In contrast, as shown in FIG. 10, for MMP2, which is a MMP whose transcription is not regulated by a xenobiotic responsive element (XRE), no decrease in the mRNA expression was observed even in the HaCaT cells incubated in the medium containing 1 μM, 10 μM and 100 μM of Fraglide-1. As demonstrated herein, Fraglide-1 selectively decreases the expression level of MMP9, which is transcriptionally regulated by a foreign body response in which a xenobiotic responsive element (XRE) is involved.

Next, the effect of Fraglide-1 on the expression of MMP9 in the protein level was examined. Specifically, HaCaT cells were suspended in a DMEM medium at the density of $1 \times 10^5$ cell/mL, and 100 μL of the thus obtained suspension was added to each well of 96-well plates so that the number of the cells in each well was 10,000 cells/well. In accordance with a conventional method, the cells were incubated for 24 hours at 37°C and 5% $CO_2$. Thereafter, the DMEM medium was removed, and 100 μL of a serum-free medium containing 0 μM or 100 μM of Fraglide-1 was added, followed by further 24 hours incubation at 37°C and 5% $CO_2$. Gelatin zymography was conducted using 10 μL of this culture supernatant.

Gelatin zymography was performed in accordance with the conventional method (https://www.abcam.com/protocols/gelatin-zymography-protocol). Specifically, proteins in the above culture supernatant were separated by SDS-PAGE using a 7.5% polyacrylamide gel containing gelatin at a final concentration of 1 mg/mL. After electrophoresis, the gel was immersed in a washing buffer containing final concentrations of 5 mM $Ca^{2+}$, 1 μM $Zn^{2+}$, and 2.5% Triton X-100, thereby SDS in the gel was removed.

The washed gel was further immersed overnight at 3°C in an incubation buffer containing final concentrations of 5 mM $Ca^{2+}$, 1 μM $Z^{n2}+$ and 1% Triton X-100 for enzymatic reaction. Gelatins in the thus obtained gel was visualized by Coomassie Blue staining. In the portion where the active-type MMP9 is present, gelatins in the gel are decomposed by the gelatinase activity of the active-type MMP9. Meanwhile, in the portion where the active-type MMP9 is not present, gelatins are not decomposed and stained blue by Coomassie Blue staining. Accordingly, the active-type MMP9 is detected as transparent bands in the gel stained blue by Coomassie Blue staining. The obtained result was shown in FIG. 11.

As shown in FIG. 11, the gelatinase activity of MMP9 was decreased by approximately 54% and 24% in the HaCaT cells incubated in the medium containing 100 μM of Fraglide-1, compared to the HaCaT cells incubated in the medium without Fraglide-1. This result indicates that the amount of MMP9 protein was decreased by Fraglide-1. This result is also consistent with the result shown in FIG. 9, which indicated that Fraglide-1 significantly decreased the mRNA expression of MMP9.

<Experiment 4-2: Comparison with Retinoic Acid and Nicotinamide>

Next, experiments were performed to inhibit MMP9 and MMP2 in HaCaT cells using retinoic acid (CAS RN: 302-79-4) and nicotinamide (CAS RN:98-92-0), which are known to be substances having anti-wrinkle effect. Briefly, HaCaT cells were made in contact with retinoic acid and nicotinamide in the same manner as in Experiment 4-1 except that retinoic acid and nicotinamide were used instead of Fraglide-1. Then, the total RNA was extracted, cDNA was prepared, and the quantitative PCR was carried out using the obtained cDNA.

The obtained results were shown in FIG. 12 and FIG. 13. In FIG. 12 and FIG. 13, the results obtained for each sample were shown as a relative value, with the mRNA expression level of MMP9 or MMP2 in the HaCaT cells incubated for 24 hours in DMEM medium containing neither retinoic acid nor nicotinamide (i.e., the non-treated HaCaT cells) set to 1. The results shown in FIG. 12 and FIG. 13 were the mean obtained from three independent experiments. The error bars represent the standard deviation (SD).

As shown in FIG. 12, the decreased expression of MMP9 was also observed in the HaCaT cells incubated in the medium containing retinoic acid ("RA" in FIG. 12. The same applies to FIG. 13) or nicotinamide ("NA" in FIG. 12. The same applies to FIG. 13). However, as shown in FIG. 13, in the HaCaT cells incubated in the medium containing retinoic acid or nicotinamide, not only the expression of MMP9 transcriptionally regulated by a xenobiotic responsive element (XRE), but also the expression of MMP2, which is not transcriptionally regulated by a xenobiotic responsive element, in other word, which is not related to a foreign body reaction in the skin, was decreased at the same time. This result indicates that the expression of many genes including MMP2, which is a matrix metalloprotease other than MMP9, is unselectively inhibited by retinoic acid and nicotinamide, which is conventionally used as an active ingredient of anti-wrinkle agents. Such unselective inhibitions of MMPs may not be preferred because it may interfere with degradation of extracellular matrix required in normal tissues.

In contrast, as shown in Experiment 4-1, Fraglide-1 selectively inhibits the expression of MMP9, which is transcriptionally regulated by a xenobiotic responsive element (XRE), but does not inhibit the expression of MMP2. This would be very advantageous because it is less likely to degrade extracellular matrix required in normal tissues. In other words, Fraglide-1 selectively inhibits the expression of MMP9 caused by a foreign body reaction in the skin triggered by various chemical substances such as environmental pollutants. Therefore, it is less likely to exhibit side effects and is particularly preferable as an active ingredient of anti-wrinkle agents. Needless to say, Fraglide-1, which selectively inhibits the expression of MMP9, is very useful not only as an active ingredient of anti-wrinkle agents but also as an active ingredient of agents for treating and/or preventing various diseases known to be associated with promoted expression of MMP9.

<Experiment 5: Effect of Fraglide-1 on TIMP-1>

As described above, it was shown that Fraglide-1 selectively inhibited the expression of matrix metalloprotease 9, which is known to be a cause of wrinkles. Then, in order to examine the effect of Fraglide-1 on other factors related to wrinkles, the effect of Fraglide-1 on the expression of TIMP-1 (Tissue Inhibitors of Metalloproteinase-1), a protein having an inhibitory effect on MMPs, was examined. It is known that collagen homeostasis is maintained in the living body by the antagonistic action of matrix metalloproteinases (MMPs), which are a collagenase, and TIMP (Tissue Inhibitors of Metalloproteinase), which has an inhibitory effect on MMPs. When the balance between the two is lost and the expression of MMPs becomes dominant, collagen is excessively degraded, causing wrinkles.

Specifically, the mRNA expression of TIMP-1 in the HaCaT cells treated with Fraglide-1 was evaluated by performing quantitative PCR using cDNA obtained in Experiment 4-1. The procedure of quantitative PCR was the same as described in Experiment 4-1 except that the primers shown in below Table 5 were used.

[Table 5]

| Gene | | Nucleotide Sequence | |
|---|---|---|---|
| GAPDH | Forward primer | 5'-AGCCACATCGCTCGAGACAC-3' | (SEQ ID. NO. 1) |
| | Reverse primer | 5'-GCCCAATACGACCAAATCC-3' | (SEQ ID. NO. 2) |
| TIMP-1 | Forward primer | 5'-AAGACCTACACTGTTGGCTGTGAG-3' | (SEQ ID. NO. 9) |
| | Reverse primer | 5'-GTCCGTCCACAAGCAATGAG-3' | (SEQ ID. NO. 10) |

The obtained results were shown in FIG. 14. In FIG 14, the results obtained for each sample were shown in relative values normalized by setting the expression level of TIMP-1 in the HaCaT cells incubated for 24 hours in DMEM medium not containing Fraglide-1 (i.e., the non-treated HaCaT cells) as 1. The results shown in FIG. 14 are the mean of values obtained from three independent experiments, and the error bars represent standard deviation (SD).

As shown in FIG. 14, the Fraglide-1 concentration-dependent increase of the expression level of TIMP-1 was observed in the HaCaT cells incubated in the medium containing 1 μM, 10 μM, and 100 μM of Fraglide-1. In particular, when the incubation was carried out in the medium containing 100 μM of Fraglide-1, the expression level of TIMP-1 was increased about 4-fold compared to the non-treated HaCaT cells. This result indicates that Fraglide-1 has the effect of inhibiting the activity of MMP9 not only by reducing the expression level of MMP9 but also by increasing an expression level of TIMP-1, which works as an inhibitor of MMP9. In other words, Fraglide-1 is useful not only as an active ingredient of an agent for inhibiting production of MMP9 but also as an active ingredient of an agent for promoting production of

TIMP-1. As shown above, Fraglide-1 exerts anti-wrinkle effects from many aspects, being considered to be very advantageous for use in improving and/or preventing wrinkles.

On the other hand, as comparison, the similar evaluation was performed for retinoic acid and nicotinamide, which are known as active ingredients of anti-wrinkle agents, using cDNA obtained in Experiment 4-2. The procedure for quantitative PCR was the same as already described in Experiment 3-1, except that the primers shown in Table 5 were used.

The obtained results were shown in FIG. 15. In FIG. 15, the results obtained for each sample were shown in relative values normalized by setting the expression level of TIMP-1 in the HaCaT cells incubated for 24 hours in DMEM medium not containing either retinoic acid and nicotinic acid amide (i.e., the non-treated HaCaT cells) as 1. The results shown in FIG 15 are the mean of values obtained from three independent experiments, and the error bars represent standard deviation (SD).

As shown in FIG. 15, an increase of the expression level of TIMP-1 was not observed in the HaCaT cells incubated with either retinoic acid or nicotinic acid amide. This result indicates that Fraglide-1, which not only decreases the expression level of MMP9 but also increases the expression of TIMP-1, is very useful as an active ingredient of anti-wrinkle agents compared to retinoic acid and nicotinic acid amide, which are conventionally used as an active ingredient of anti-wrinkle agents.

<Experiment 6: Safety Evaluation of Fraglide-1>

The safety of Fraglide-1 was examined by a viable cell counting method using a water-soluble tetrazolium salt (WST-1). Specifically, HaCaT cells were suspended in DMEM medium at the concentration of $1 \times 10^5$ cells/mL. The obtained cell suspension was seeded in a white 96-well plate (ThermoFisher Scientific; Catalogue No. 136102) in an amount of 100 μL per well so that the number of cells per well was 10,000 cells/well. After 24 hours incubation at 37°C and 5% $CO_2$ in accordance with a conventional method, DMEM medium was removed and then either 100 μL of DMEM medium containing Fraglide-1 at a predetermined concentration or, as a control, 100 μL of DMEM medium not containing Fraglide-1 was added, followed by further incubation for 24 hours at 37°C and 5% $CO_2$. After the incubation for 24 hours, the number of viable cells was measured by using a premix reagent containing WST-1 ("Premix WST-1 Cell Proliferation Assay System", Catalogue No. MK400, Takara Bio Inc., Shiga, Japan) in accordance with the procedure described in Experiment 1-2. The obtained results were shown in FIG. 16. The results shown in FIG. 16 are the mean of values obtained from three independent experiments, and the error bars represent standard error (SE).

As shown in FIG. 16, the cell viability was more than 50% even in the HaCaT cells incubated in the DMEM medium containing 500 μM of Fraglide-1, indicating that LC50 (Median Lethal Concentration) of Fraglide-1 against HaCaT cells is at least over 500 μM. This result indicates that Fraglide-1 is a highly safe substance with low cytotoxicity. Therefore, Fraglide-1 may be advantageously used as an ingredient of a wide range of products, including pharmaceuticals, cosmetics, health foods, functional foods, daily necessities, and the like.

Various modifications that can be easily achieved by those skilled in the art without departing from the scope of the claims also fall within the scope of the invention. The contents of the articles, patent laid-open publications, patent publications, and the like specified in the present description shall be cited by incorporation in their entity.

Formulation examples of the agent in accordance with the present invention are shown below, but the present invention is not limited by these examples.

**<Formulation Example 1: Shampoo>**

| | |
|---|---|
| Water | 87.20% by mass |
| TEA-Lauroyl Methylaminopropionate | 6.00% by mass |
| Lauryl Betaine | 3.00% by mass |
| Polyquaternium-10 | 0.30% by mass |
| Sodium hydrogen carbonate | 0.02% by mass |
| Sodium lactate | 0.20% by mass |
| Cocamide Methyl MEA | 1.00% by mass |
| Cocamide DEA | 1.00% by mass |
| Lactic Acid | 0.28% by mass |
| Etidronic Acid | 0.06% by mass |
| Pentasodium Pentetate | 0.04% by mass |

(continued)

| | |
|---|---|
| Sodium Benzoate | 0.40% by mass |
| Fragrance | 0.30% by mass |
| Fraglide-1 | 0.20% by mass |

**<Formulation Example 2: Hair Treatment>**

| | |
|---|---|
| Water | 86.73% by mass |
| Glycerin | 5.00% by mass |
| Cetanol | 3.00% by mass |
| Stearyl Alcohol | 1.50% by mass |
| Behentrimonium Methosulfate | 1.50% by mass |
| Amodimethicone | 0.50% by mass |
| Dimethicone | 1.00% by mass |
| Hydroxyethylcellulose | 0.05% by mass |
| Phenoxyethanol | 0.32% by mass |
| Fragrance | 0.30% by mass |
| Fraglide-1 | 0.10% by mass |

**<Formulation Example 3: Hair Milk (Leave-on Treatment)>**

| | |
|---|---|
| Water | 93.85% by mass |
| Dimethiconol | 2.50% by mass |
| Polyacrylamide | 1.20% by mass |
| C13-14 Isoparaffin | 0.72% by mass |
| Sodium Sulfate | 0.02% by mass |
| Hydroxyethylcellulose | 0.01% by mass |
| Sodium Lauryl Sulfate | 0.08% by mass |
| Sodium Laureth Sulfate | 0.08% by mass |
| Polysorbate 20 | 0.50% by mass |
| Laureth-7 | 0.18% by mass |
| Phenoxyethanol | 0.33% by mass |
| Methylparaben | 0.01% by mass |
| Ethylparaben | 0.30% by mass |
| Propylparaben | 0.05% by mass |
| Butylparaben | 0.02% by mass |
| Fragrance | 0.10% by mass |
| Fraglide-1 | 0.05% by mass |

**<Formulation Example 4: Skin Milk (Milky Lotion)>**

| | |
|---|---|
| Water | 82.89% by mass |
| EDTA-2Na | 0.01% by mass |
| Carbomer | 0.20% by mass |

(continued)

| | |
|---|---|
| Glycerin | 3.00% by mass |
| 1,3-Butylene glycol | 7.00% by mass |
| Phenoxyethanol | 0.30% by mass |
| Methylparaben | 0.20% by mass |
| Potassium Hydroxide | 0.06% by mass |
| Ceteareth-13 | 1.00% by mass |
| Sorbitan Sesquioleate | 0.30% by mass |
| Mineral Oil | 5.00% by mass |
| Fraglide-1 | 0.04% by mass |

**<Formulation Example 5: Facial Wash>**

| | |
|---|---|
| Water | 37.16% by mass |
| Myristic acid | 20.00% by mass |
| Palmitic acid | 4.00% by weight |
| Lauric acid | 2.00% by weight |
| Stearic acid | 1.00% by mass |
| Potassium Hydroxide | 5.80% by mass |
| Polyglyceryl Lauryl Ether | 3.00% by mass |
| Glyceryl Stearate | 1.00% by mass |
| Glycol Distearate | 2.00% by mass |
| Glycerin | 20.00% by mass |
| 1,3-Butylene Glycol | 1.00% by mass |
| Lauryl Betaine | 3.00% by mass |
| Fraglide-1 | 0.04% by mass |

**<Formulation Example 6: Body Soap>**

| | |
|---|---|
| Water | 65.50% by mass |
| Lauric Acid | 5.50% by mass |
| Myristic Acid | 10.00% by mass |
| Stearic Acid | 1.00% by mass |
| Lauryl Betaine | 3.50% by mass |
| Polyglyceryl Lauryl Ether | 2.00% by mass |
| Propylene Glycol | 5.00% by mass |
| Glycol Distearate | 2.00% by mass |
| Acrylates/Alkyl Acrylate Crosspolymer | 0.10% by mass |
| Sodium Hydroxide | 4.00% by mass |
| Sodium Chloride | 1.00% by mass |
| Fragrance | 0.30% by mass |
| Fraglide-1 | 0.10% by mass |

**<Formulation Example 7: Body Milk>**

| | |
|---|---|
| Water | 85.60% by mass |

(continued)

| | |
|---|---|
| EDTA-2Na | 0.01% by mass |
| Carbomer | 0.20% by mass |
| Hydroxyethylcellulose | 0.10% by mass |
| 1,3-Butylene Glycol | 8.00% by mass |
| Phenoxyethanol | 0.30% by mass |
| Methylparaben | 0.20% by mass |
| Potassium Hydroxide | 0.17% by mass |
| Polyglyceryl Decaisostearate | 3.00% by mass |
| Olive Oil | 2.00% by mass |
| Acrylates/Alkyl Acrylate Crosspolymer | 0.20% by mass |
| Fragrance | 0.20% by mass |
| Fraglide-1 | 0.02% by mass |

**<Formulation Example 8: Bath Milk>**

| | |
|---|---|
| Water | 24.37% by mass |
| Olive Oil | 45.00% by mass |
| 1,3-Butylene Glycol | 8.00% by mass |
| Polyglyceryl Oleate | 5.00% by mass |
| Polyglyceryl Decaisostearate | 2.00% by mass |
| Bentonite | 15.00% by mass |
| Methylparaben | 0.20% by mass |
| Citric Acid/Sodium Citrate | 0.40% by mass |
| Fraglide-1 | 0.03% by mass |

**Industrial Applicability**

In modern society, where various chemical substances are used in various products around us, it is extremely important to know the effects of chemical substances on the skin. The evaluation method in accordance with one aspect of the present invention, which enables easy and quick evaluation of an ability of various test samples to trigger a foreign body reaction in the skin, is expected to be useful in a wide range of industrial fields, such as cosmetics, pharmaceuticals, and food products, etc., regardless of the field. Meanwhile, the agent for inhibiting a foreign body reaction in the skin in accordance with one aspect of the present invention, may be comprised in many products to keep the skin healthy, for example, for the purpose of preventing and/or treating atopic dermatitis and preventing and/or improving wrinkles. By the screening method in accordance with one aspect of the present invention, a substance having a property of inhibiting a foreign body reaction in the skin, which may be used as an active ingredient in such an agent, may be easily and rapidly screened. As discussed above, the industrial applicability of the present invention is huge.

**Claims**

**1.** A method for evaluating an ability of a test sample to trigger a foreign body reaction in the skin, comprising:

(1) a step of making a solution containing said test sample in contact with a keratinocyte transformed by a reporter vector comprising an enhancer sequence comprising a xenobiotic responsive element (XRE) and a reporter sequence encoding a reporter protein at downstream of said enhancer sequence;
(2) a step of measuring an expression level of said reporter protein in said keratinocyte that is made in contact with said solution containing said test sample; and
(3) a step of evaluating an ability of said test sample to trigger a foreign body reaction in the skin based on the measured expression level of said reporter protein.

2. A method for screening a substance having an ability to inhibit a foreign body reaction in the skin, comprising:

(1) a step of making a test solution containing a candidate substance and a reference solution without said candidate substance in contact with a keratinocyte transformed by a reporter vector comprising an enhancer sequence comprising a xenobiotic responsive element (XRE) and a reporter sequence encoding a reporter protein at downstream of said enhancer sequence;
(2) a step of measuring an expression level of said reporter protein in said keratinocyte that is made in contact with said test solution and in said keratinocyte that is made in contact with said reference solution, and
(3) a step of evaluating said test candidate as having an ability to inhibit a foreign body reaction in the skin, when said expression level of said reporter protein in said keratinocyte that is made in contact with said test solution is smaller than said expression level of said reporter protein in said keratinocyte that is made in contact with said reference solution.

3. The method according to claim 2, wherein said test solution and said reference solution contain a substance that triggers a foreign body reaction in the skin.

4. An agent for inhibiting an expression of a gene transcribed with involvement of a xenobiotic responsive element (XRE), comprising 5-hydroxy-4-phenyl-2(5H)butanolide and/or its salt.

5. An agent for inhibiting production of matrix metalloprotease 9, comprising 5-hydroxy-4-phenyl-2(5H)butanolide and/or its salt.

6. The agent according to claim 5, which is for use in preventing and/or improving wrinkles.

7. An agent for inhibiting production of artemin, comprising 5-hydroxy-4-phenyl-2(5H)butanolide and/or its salt.

8. The agent according to claim 7, which is for use in preventing and/or treating atopic dermatitis.

9. A pharmaceutical, quasi-drug, cosmetic product, daily necessity, or food product comprising the agent according to any one of claims 4 to 8.

10. The cosmetic product according to claim 9, which is in the form of skin lotion, milky lotion, cream, serum, cleansing, beauty mask, face wash, soap, makeup base, foundation, concealer, face powder, lipstick, blush, eyeshadow, eyeliner, eyebrow, mascara, body soap, body milk, body lotion, body cream, body oil, body powder, sunscreen, sun oil, deodorant spray, bleaching cream, depilatory cream, hand cream, shampoo, rinse, conditioning shampoo, dry shampoo, hair treatment, conditioner, hair milk, hair cream, hair oil, hair mist, tonic, hair foam, hair gel, wax, pomade, hair liquid, wave agent, hair color, bleach, color rinse, hair restorer, hair growth stimulant, toothpaste, mouthwash, gargle, bath milk, bath salt, bath oil, perfume, or cologne.

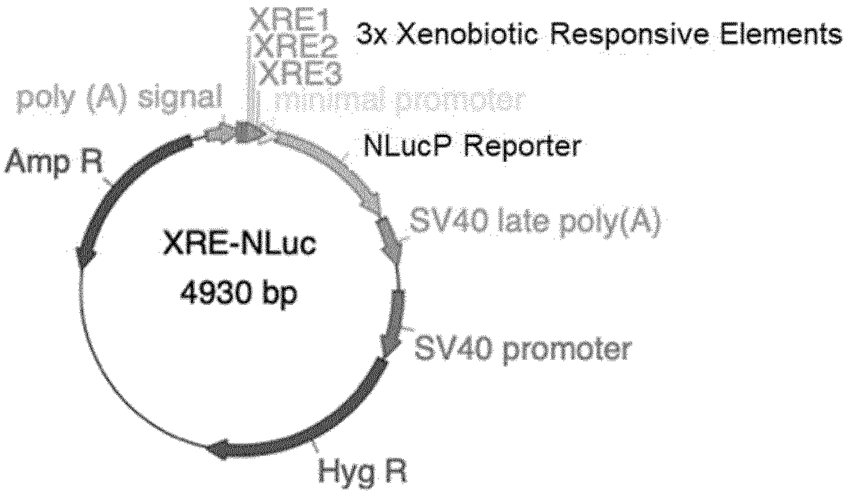
XRE1
XRE2
XRE3
3x Xenobiotic Responsive Elements
poly (A) signal
minimal promoter
NLucP Reporter
Amp R
SV40 late poly(A)
XRE-NLuc
4930 bp
SV40 promoter
Hyg R

FIG. 1

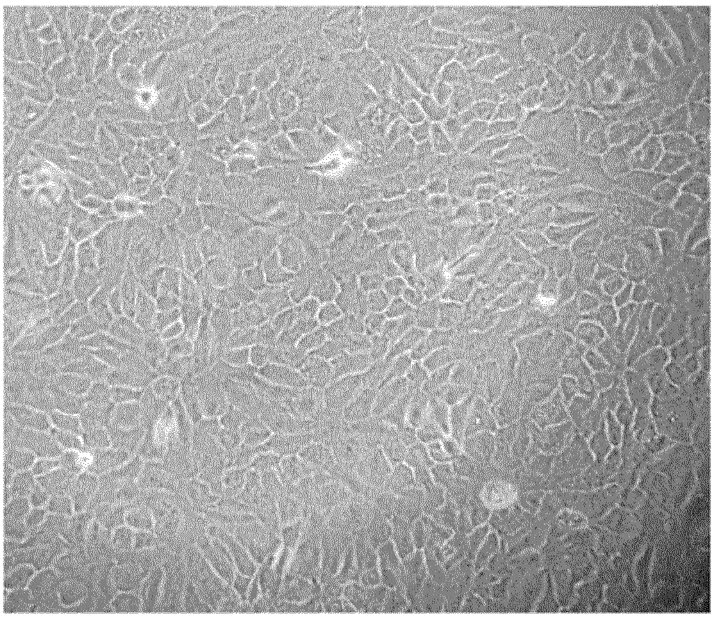

FIG. 2

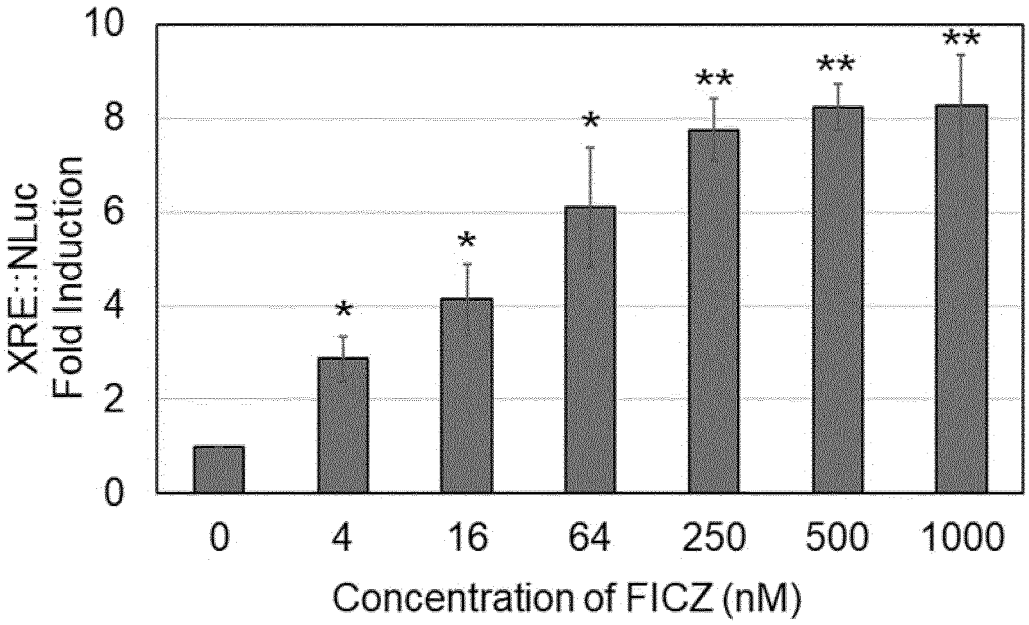
10
8
6
4
2
0
XRE::NLuc
Fold Induction
*
*
*
**
**
**
0
4
16
64
250
500
1000
Concentration of FICZ (nM)

FIG. 3

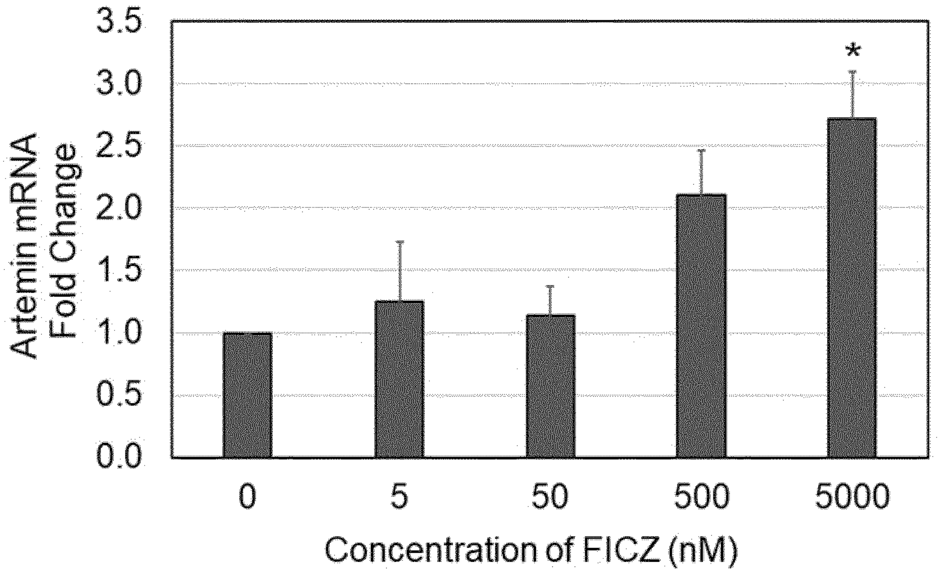
3.5
3.0
2.5
2.0
1.5
1.0
0.5
0.0
Artemin mRNA
Fold Change
*
0
5
50
500
5000
Concentration of FICZ (nM)

FIG. 4

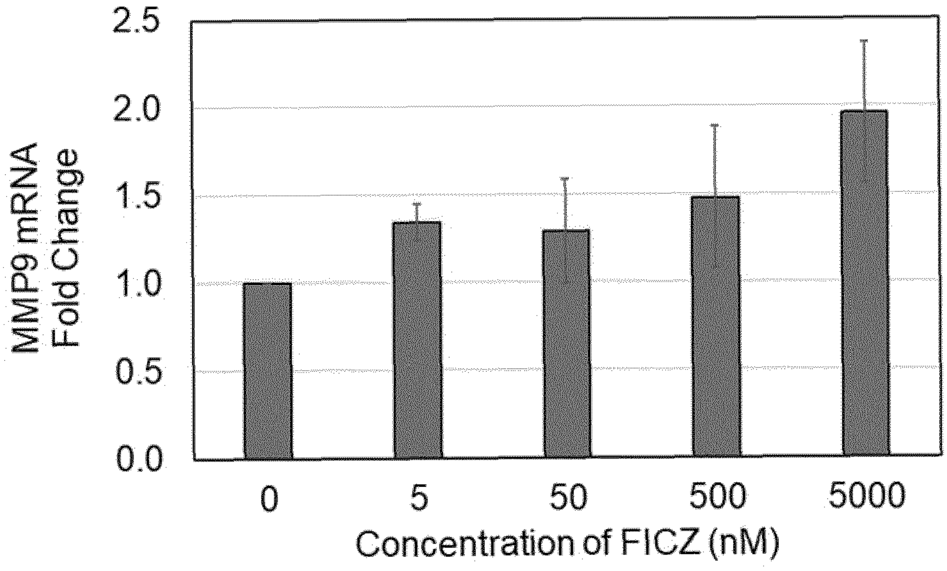
2.5
2.0
1.5
1.0
0.5
0.0
MMP9 mRNA
Fold Change
0
5
50
500
5000
Concentration of FICZ (nM)

FIG. 5

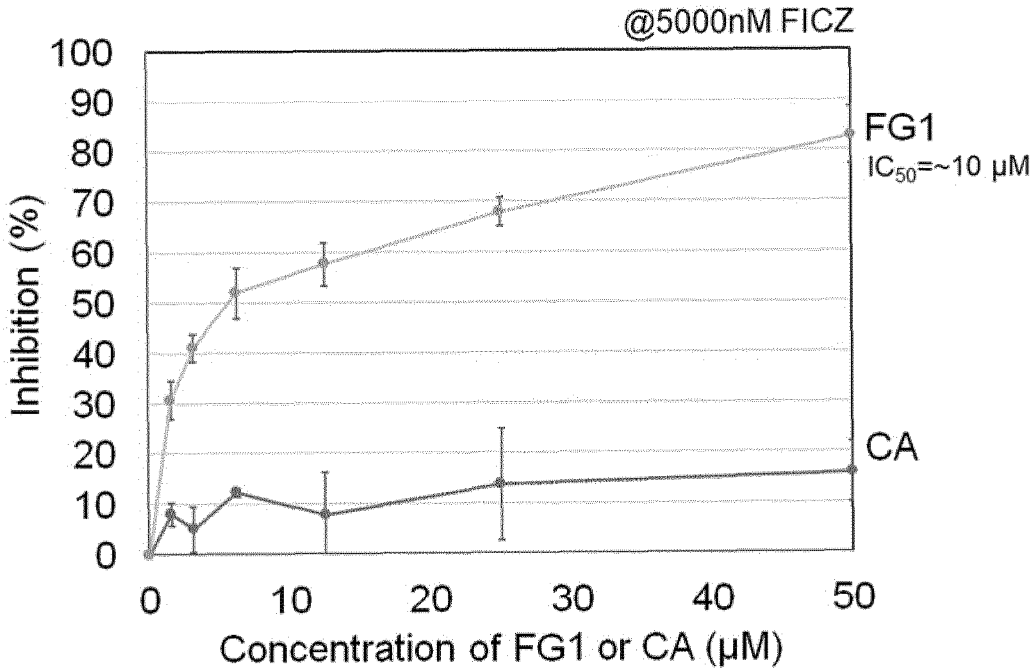
@5000nM FICZ
100
90
80
70
60
50
40
30
20
10
0
Inhibition (%)
FG1
IC50=~10 μM
CA
0
10
20
30
40
50
Concentration of FG1 or CA (μM)

FIG. 6

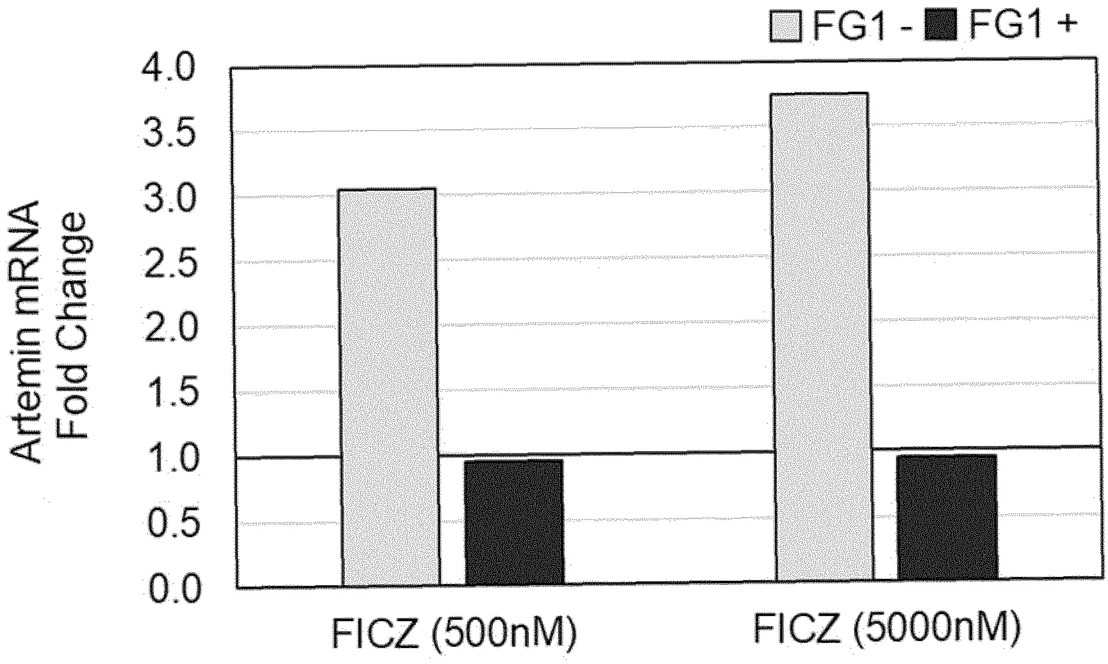
FG1 -
FG1 +
Artemin mRNA Fold Change
4.0
3.5
3.0
2.5
2.0
1.5
1.0
0.5
0.0
FICZ (500nM)
FICZ (5000nM)

FIG. 7

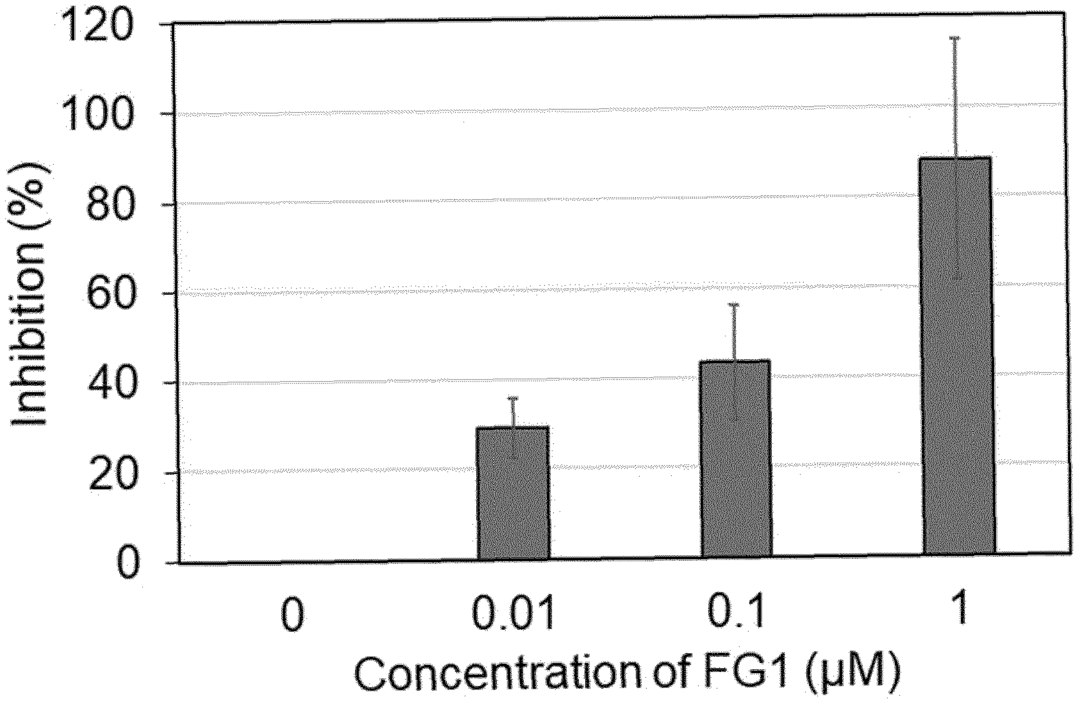
120
100
80
60
40
20
0
Inhibition (%)
0
0.01
0.1
1
Concentration of FG1 (µM)

FIG. 8

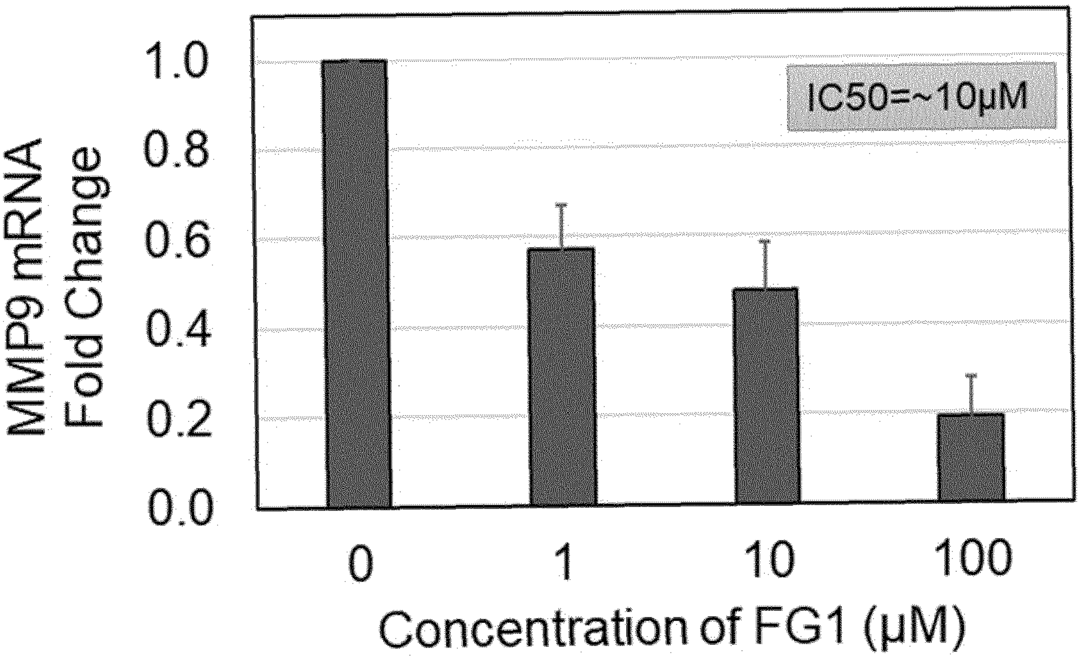
IC50=~10μM
MMP9 mRNA Fold Change
1.0
0.8
0.6
0.4
0.2
0.0
0
1
10
100
Concentration of FG1 (μM)

FIG. 9

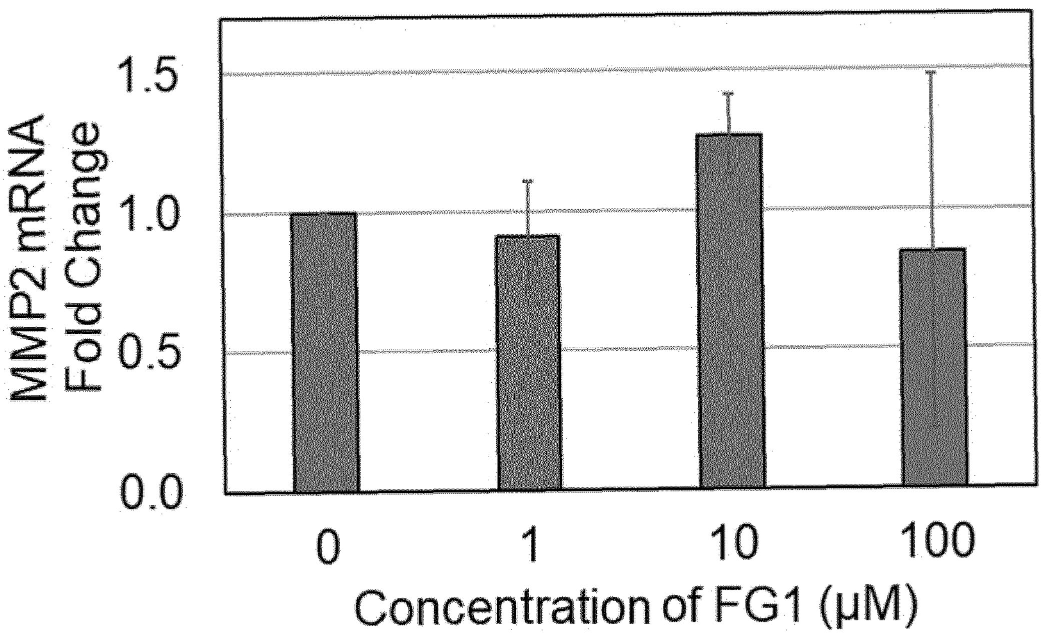
MMP2 mRNA Fold Change
1.5
1.0
0.5
0.0
0
1
10
100
Concentration of FG1 (μM)

FIG. 10

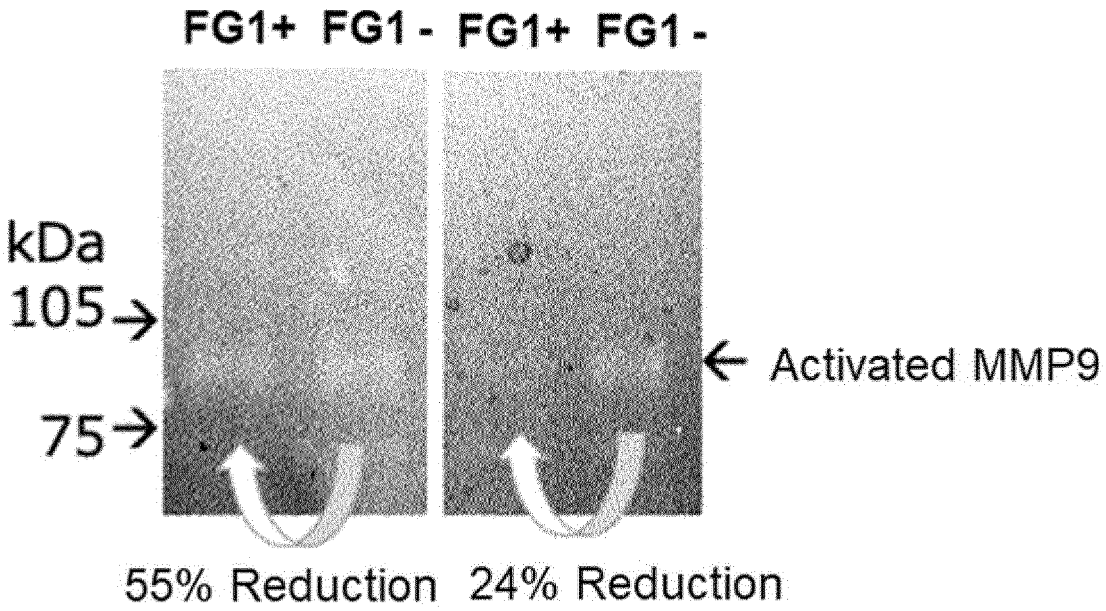
FG1+ FG1 - FG1+ FG1 -
kDa
105
75
Activated MMP9
55% Reduction
24% Reduction

FIG. 11

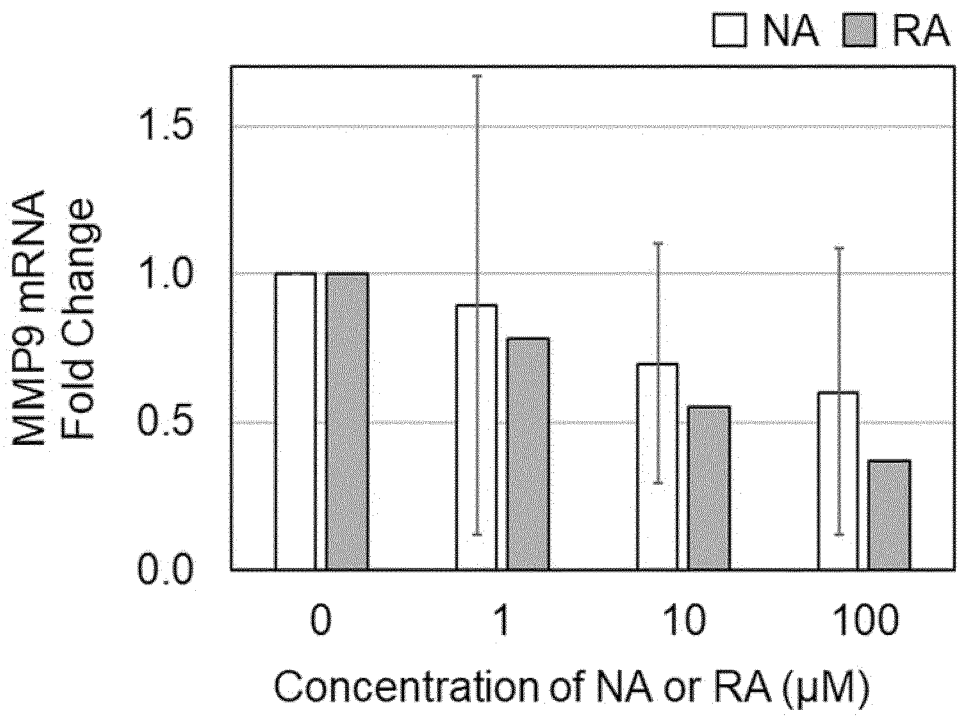
NA
RA
MMP9 mRNA Fold Change
1.5
1.0
0.5
0.0
0
1
10
100
Concentration of NA or RA (μM)

FIG. 12

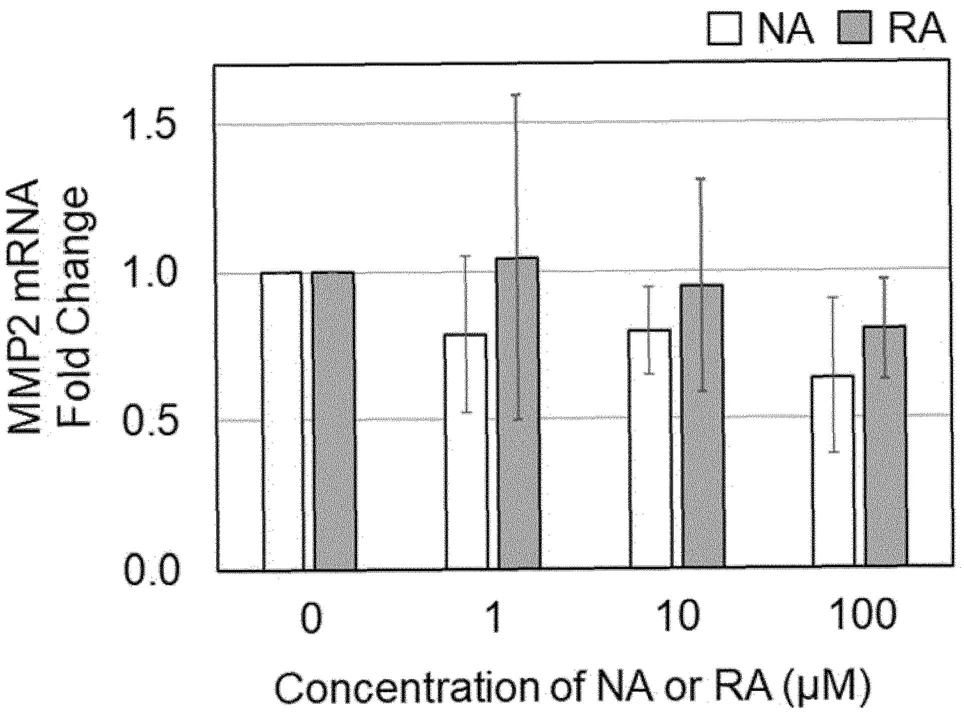
NA
RA
1.5
1.0
0.5
0.0
MMP2 mRNA
Fold Change
0
1
10
100
Concentration of NA or RA (μM)

FIG. 13

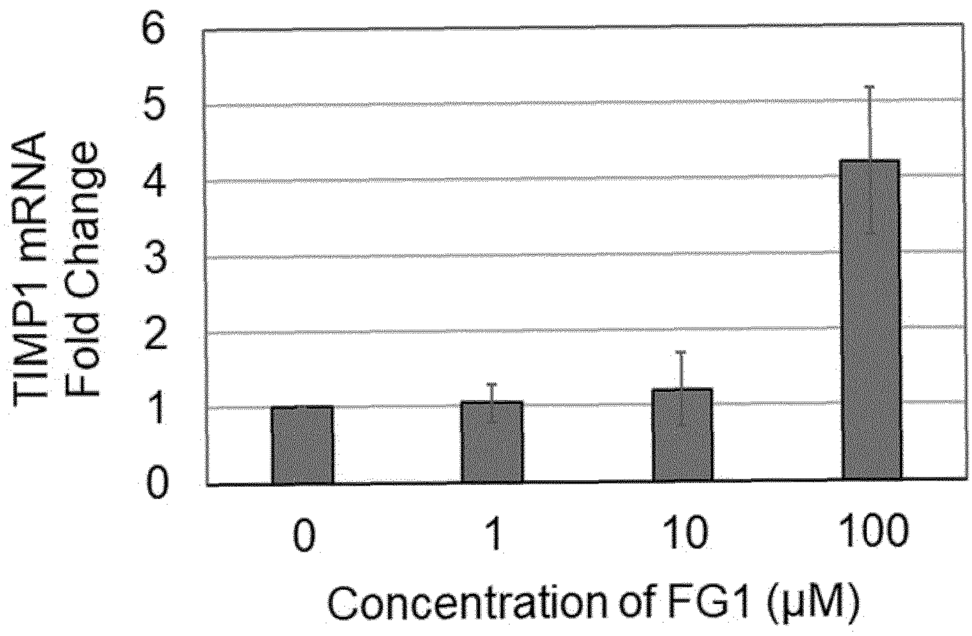
6
5
4
3
2
1
0
TIMP1 mRNA
Fold Change
0
1
10
100
Concentration of FG1 (μM)

FIG. 14

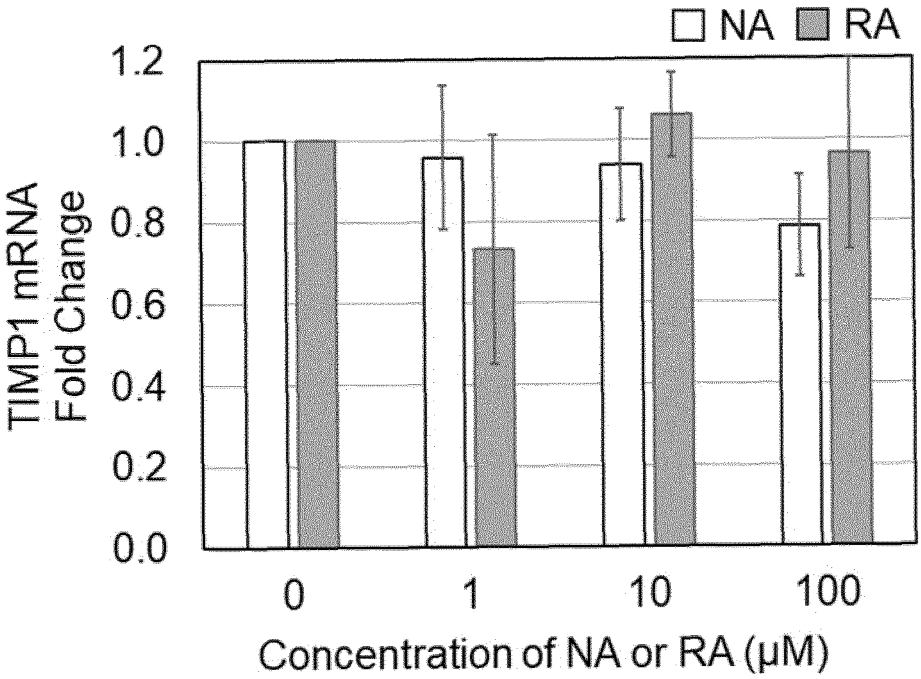
NA
RA
1.2
1.0
0.8
0.6
0.4
0.2
0.0
TIMP1 mRNA
Fold Change
0
1
10
100
Concentration of NA or RA (μM)

FIG. 15

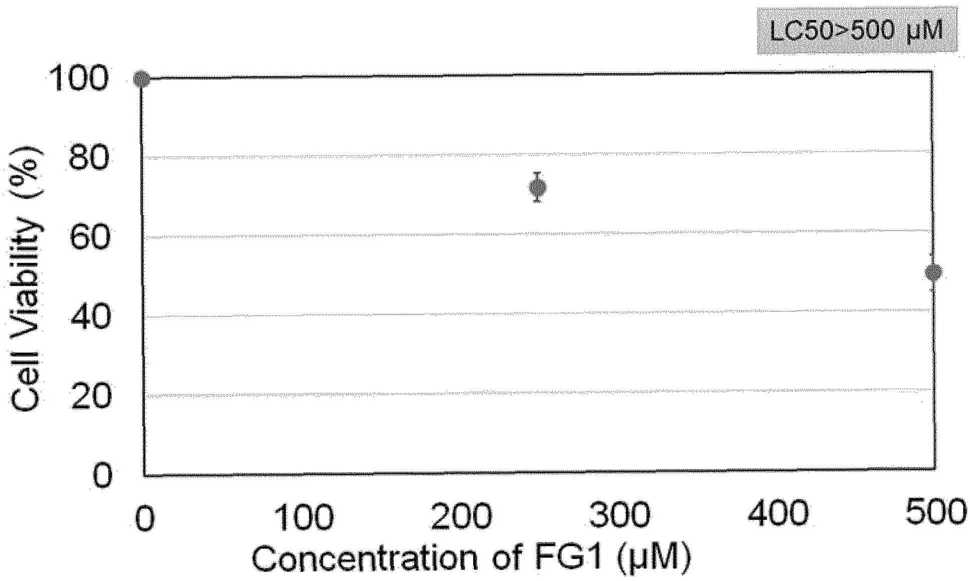
LC50>500 μM
100
80
60
40
20
0
Cell Viability (%)
0
100
200
300
400
500
Concentration of FG1 (μM)

FIG. 16

**INTERNATIONAL SEARCH REPORT**

International application No. **PCT/JP2022/006379**

**A. CLASSIFICATION OF SUBJECT MATTER**

***A61P 37/08***(2006.01)i; ***A61P 43/00***(2006.01)i; ***A61P 17/00***(2006.01)i; ***A61Q 19/08***(2006.01)i; ***A61K 8/49***(2006.01)i; ***C12N 15/55***(2006.01)i; ***C12N 15/63***(2006.01)i; ***C12Q 1/6851***(2018.01)i; ***C12Q 1/686***(2018.01)i; ***C12Q 1/6897***(2018.01)i; ***G01N 33/15***(2006.01)i; ***G01N 33/50***(2006.01)i; ***G01N 33/53***(2006.01)i; ***G01N 33/68***(2006.01)i; ***A61K 31/365***(2006.01)i

FI: G01N33/15 Z; A61K8/49; A61K31/365; A61P17/00; A61P37/08; A61P43/00 111; A61Q19/08; C12N15/55; C12N15/63 Z; C12Q1/6851 Z; C12Q1/686 Z; C12Q1/6897 Z; G01N33/50 Q ZNA; G01N33/50 Z; G01N33/53 M; G01N33/68

According to International Patent Classification (IPC) or to both national classification and IPC

**B. FIELDS SEARCHED**

Minimum documentation searched (classification system followed by classification symbols)

A61P37/08; A61P43/00; A61P17/00; A61Q19/08; A61K8/49; C12N15/55; C12N15/63; C12Q1/6851; C12Q1/686; C12Q1/6897; G01N33/15; G01N33/50; G01N33/53; G01N33/68; A61K31/365

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KATO, K. et al. Establishing a method alternative to animal testing to evaluate the induction of atopic dermatitis. ALTERNATIVES TO ANIMAL TESTING AND EXPERIMENTATION. 31 December 2020, vol. 25/supplement, p. 121<br>in particular, paragraphs [0002]-[0004] | 1-3 |
| Y | 生田統吾、川尻要, 表皮角化細胞におけるXREを介した遺伝子発現調節の解析, 生化学, 25 August 2001, vol. 73/no. 8, p. 720<br>in particular, [purpose and method], [result], (SEIKAGAKU), non-official translation (IKUTA, Togo. KAWAJIRI, Kaname. Analysis of gene expression regulation through XRE in epidermal keratinocytes.) | 1-3 |
| Y | HIDAKA, T. et al. The aryl hydrocarbon receptor AhR links atopic dermatitis and air pollution via induction of the neurotrophic factor artemin. NATURE IMMUNOLOGY. 26 November 2016, vol. 18/no. 1, pp. 64-76<br>abstract, results, discussion, fig. 1-7 | 1-3 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 April 2022** | **10 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/006379

C. DOCUMENTS CONSIDERED TO BE RELEVANT

| Category* | Citation of document, with indication, where appropriate, of the relevant passages | Relevant to claim No. |
|---|---|---|
| Y | WO 2011/068166 A1 (SHISEIDO COMPANY, LTD.) 09 June 2011 (2011-06-09) paragraphs [0036]-[0072] | 2-3 |
| Y | JP 2017-000136 A (MIKIMOTO SEIYAKU KK) 05 January 2017 (2017-01-05) paragraphs [0005]-[0015] | 2-3 |
| A | JP 2004-267090 A (JENOKKUSU SOYAKU KENKYUSHO KK) 30 September 2004 (2004-09-30) entire text, all drawings | 1-10 |
| A | JP 2004-135662 A (FRONTIER SCIENCE CO LTD) 13 May 2004 (2004-05-13) entire text, all drawings | 1-10 |
| A | JP 2018-095592 A (KOJUN JAPAN CO LTD) 21 June 2018 (2018-06-21) entire text, all drawings | 1-10 |

Form PCT/ISA/210 (second sheet) (January 2015)

INTERNATIONAL SEARCH REPORT

International application No.
**PCT/JP2022/006379**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**

International application No.
**PCT/JP2022/006379**

**Box No. III Observations where unity of invention is lacking (Continuation of item 3 of first sheet)**

This International Searching Authority found multiple inventions in this international application, as follows:

Document 1: 生田統吾、川尻要, 表皮角化細胞におけるXREを介した遺伝子発現調節の解析, 生化学, 25 August 2001, vol. 73/no. 8, p. 720, (SEIKAGAKU), non-official translation (IKUTA, Togo. KAWAJIRI, Kaname. Analysis of gene expression regulation through XRE in epidermal keratinocytes.)
Document 2: JP 2018-095592 A (KOJUN JAPAN CO LTD) 21 June 2018 (2018-06-21) (Family: none)

(Invention 1) Claims 1-3
The inventions in claims 1-3 share the common technical feature of a method for searching for a substance related to a xenobiotic response reaction in the skin, the method comprising: (1) a step for bringing keratinocytes, which are transformed with a reporter vector having an enhancer sequence including a xenobiotic response element (XRE) and a sequence disposed downstream thereof and encoding a reporter protein, into contact with a solution containing the test sample; (2) a step for measuring the expression level of the reporter protein in the keratinocytes brought into contact with the solution containing the test sample; and (3) a step for evaluating the influence of the test sample on a xenobiotic response reaction in the skin, on the basis of the measured expression level of the reporter protein, and are thus classified as invention 1.

(Invention 2) Claims 4-6, and parts referring to claims 4-6 in claims 9-10
The invention in independent claim 4 shares, with claim 1 classified as invention 1, the common technical feature of a "xenobiotic response element (XRE)", but said technical feature does not make a contribution over the prior art in light of the disclosure of document 1 (see entire text) and thus cannot be said to be a special technical feature. Moreover, there are no other same or corresponding special technical features between these inventions.
Further, claims 4-6, and parts referring to claims 4-6 in claims 9-10 are not dependent on claim 1. Furthermore, claims 4-6, and parts referring to claims 4-6 in claims 9-10 are not substantially identical to or similarly closely related to any of the claims classified as invention 1.
In addition, claims 4-6, and parts referring to claims 4-6 in claims 9-10 share the common technical feature of an "agent for suppressing the expression of genes that are transcribed with the involvement of a xenobiotic response element (XRE), the agent containing 5-hydroxy-4-phenyl-2(5H)butanolide and/or a salt thereof", and are thus classified as invention 2.

(Invention 3) Claims 7-8, and parts referring to claims 7-8 in claims 9-10
The invention in independent claim 7 does not share a common technical feature with claim 1.
Meanwhile, claim 7 shares, with claim 4, the common technical feature of "5-hydroxy-4-phenyl-2(5H)butanolide", but said technical feature does not make a contribution over the prior art in light of the disclosure of document 2 (in particular, paragraphs [0015]-[0025]) and thus cannot be said to be a special technical feature. Moreover, there are no other same or corresponding special technical features between these inventions.
Further, claims 7-8, and parts referring to claims 7-8 in claims 9-10 are not dependent on claim 1 or 4, and are not substantially identical to or similarly closely related to any of the claims classified as invention 1 or 2.
In addition, claims 7-8, and parts referring to claims 7-8 in claims 9-10 share the common technical feature of an "agent for suppressing the production of artemin, the agent containing 5-hydroxy-4-phenyl-2(5H)butanolide and/or a salt thereof", and are thus classified as invention 3.

Form PCT/ISA/210 (continuation of first sheet) (January 2015)

**INTERNATIONAL SEARCH REPORT**

International application No.
**PCT/JP2022/006379**

**Box No. III Observations where unity of invention is lacking (Continuation of item 3 of first sheet)**

1. ☑ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2022/006379**

| Patent document cited in search report | Publication date (day/month/year) | Patent family member(s) | Publication date (day/month/year) |
|---|---|---|---|
| WO 2011/068166 A1 | 09 June 2011 | EP 2508605 A1<br>paragraphs [0036]-[0073]<br>CN 102648278 A<br>KR 10-2012-0112445 A<br>US 2012/0302649 A1 | |
| JP 2017-000136 A | 05 January 2017 | (Family: none) | |
| JP 2004-267090 A | 30 September 2004 | (Family: none) | |
| JP 2004-135662 A | 13 May 2004 | (Family: none) | |
| JP 2018-095592 A | 21 June 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016006548 A
- JP H6211825 B
- JP H11152280 B
- JP 2011178747 A
- JP 2020504719 PCT
- JP 2015017048 A


### Non-patent literature cited in the description


- **B. MAGNUSSON ; A.M. KLIGMAN.** *Journal of Investigative Dermatology,* 1969, vol. 52, 268-276
- **D. A. BASKETTER et al.** *Food and Chemical Toxicology,* 2002, vol. 40, 593-598
- **SEBASTIAN HOFFMANN et al.** *Critical Reviews in Toxicology,* 2018, vol. 48, 344-358
- **TAKANORI HIDAKA et al.** *Nature Immunology,* 2017, vol. 18, 64-73
- **E. DUPONT ; J. GOMEZ ; D. BILODEAU.** *International Journal of Cosmetic Science,* 2013, vol. 35, 224-232
- **MICHAEL S. DENISON ; SCOTT R. NAGY.** *Annual Review of Pharmacology and Toxicology,* 2003, vol. 43, 309-334
- *CHEMICAL ABSTRACTS,* 17922-91-7
- *CHEMICAL ABSTRACTS,* 700-06-1
- *CHEMICAL ABSTRACTS,* 448906-42-1
- *CHEMICAL ABSTRACTS,* 479-41-4
- **AYDAN H. YATMAZ et al.** *Journal of Oleo Science,* 2017, vol. 66, 1381-1386
- **SHINJI INOMATA et al.** *Journal of Investigative Dermatology,* 2003, vol. 120, 128-134
- **TAKASHI KOBAYASHI.** *Inflammation and Regeneration,* 2004, vol. 24, 578-583
- *CHEMICAL ABSTRACTS,* 172922-91-7
- **ANETA NOVOTNA et al.** *Environ. Sci. Technol.,* 2011, vol. 45 (23), 10133-10139
- **HIDEO SATSU et al.** *Cytotechnology,* 2015, vol. 67, 621-632
- *CHEMICAL ABSTRACTS,* 302-79-4
- *CHEMICAL ABSTRACTS,* 98-92-0